# EUROPÄISCHE PATENTANMELDUNG

(11) **EP 3 951 791 A2**
(43) Veröffentlichungstag der Anmeldung: **09.02.2022**
(21) Anmeldenummer: 21184230.7
(22) Anmeldetag: 07.07.2021
(51) Int. Cl.: G16H 40/63, G16H 20/40

(54) **DENTALSYSTEM**

(30) Priorität: 14.07.2020 DE 102020118554
(71) Anmelder: Renfert GmbH, 78247 Hilzingen (DE)
(72) Erfinder: Egelhof, Joachim, 73655 Plüderhausen (DE)
(74) Vertreter: Daub, Thomas

(57) **Zusammenfassung**

Die Erfindung geht aus von einem Dentalsystem mit zumindest einem Dentalgerät (12, 12', 12", 12'", 12""), welches zumindest eine Steuer- und/oder Regeleinheit (14) aufweist, die zu einer Steuerung und/oder Regelung zumindest eines Prozesses des Dentalgeräts (12, 12', 12", 12'", 12"") vorgesehen ist, mit zumindest einer mit dem Dentalgerät (12, 12', 12", 12'", 12"") gekoppelten Kommunikationseinheit (16) und mit zumindest einem Bedienerendgerät (18), welches über die Kommunikationseinheit (16) zu einem Datenaustausch mit dem Dentalgerät (12, 12', 12", 12'", 12"") vorgesehen ist.

Es wird vorgeschlagen, dass das Bedienerendgerät (18) dazu vorgesehen ist, dem Bediener (20) zugeordnete Profilparameter für das Dentalgerät (12, 12', 12", 12'", 12"") zu erfassen.

## Beschreibung

### Stand der Technik

Die Erfindung betrifft ein Dentalsystem.

Es ist bereits ein Dentalsystem mit zumindest einem Dentalgerät, welches zumindest eine Steuer- und/oder Regeleinheit aufweist, die zu einer Steuerung und/oder Regelung zumindest eines Prozesses des Dentalgeräts vorgesehen ist, mit zumindest einer mit dem Dentalgerät gekoppelten Kommunikationseinheit und mit zumindest einem Bedienerendgerät, welches über die Kommunikationseinheit zu einem Datenaustausch mit dem Dentalgerät vorgesehen ist, vorgeschlagen worden.

Die Aufgabe der Erfindung besteht insbesondere darin, eine gattungsgemäße Vorrichtung mit verbesserten Eigenschaften hinsichtlich eines Komforts sowie einer Bedieneranpassung bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einem Dentalsystem mit zumindest einem Dentalgerät, welches zumindest eine Steuer- und/oder Regeleinheit aufweist, die zu einer Steuerung und/oder Regelung zumindest eines Prozesses des Dentalgeräts vorgesehen ist, mit zumindest einer mit dem Dentalgerät gekoppelten Kommunikationseinheit und mit zumindest einem Bedienerendgerät, welches über die Kommunikationseinheit zu einem Datenaustausch mit dem Dentalgerät vorgesehen ist.

Es wird vorgeschlagen, dass das Bedienerendgerät dazu vorgesehen ist, dem Bediener zugeordnete Profilparameter für das Dentalgerät zu erfassen. Vorzugsweise ist das Bedienerendgerät dazu vorgesehen, dem Bediener zugeordnete Profilparameter abzurufen und insbesondere davon abhängige Prozessparameter auf das Dentalgerät zu übertragen. Die dem Bediener zugeordneten Profilparameter können dabei insbesondere sowohl direkt auf dem Bedienerendgerät hinterlegt sein als auch auf einer Datenbank eines externen oder internen Servers.

Hierbei ist es denkbar, dass die Kommunikationseinheit ein Teil des Dentalgeräts ausbildet und/oder in das Dentalgerät integriert ist und/oder von einer separaten, mit dem Dentalgerät koppelbaren Einheit gebildet ist. Dabei wäre insbesondere denkbar, dass die Kommunikationseinheit ein Teil einer universellen Kommunikationsvorrichtung ausbildet, die zumindest eine direkt, mechanisch mit der Datenschnittstelle des Dentalgeräts koppelbare Datenschnittstelle aufweist. Es wäre insbesondere denkbar, dass die Kommunikationseinheit ein Teil eines Datensticks bildet, welcher eine Kommunikation des Dentalgeräts ermöglicht.

Unter einem "Dentalgerät" soll in diesem Zusammenhang insbesondere ein Gerät zu einer speziellen Anwendung in einer Zahnarztpraxis und/oder in einem Dentallabor verstanden werden. Vorzugsweise soll darunter insbesondere ein Gerät zu einer Herstellung und/oder Bearbeitung von Zahnkomponenten und/oder Zahnersatzkomponenten und/oder ein Gerät zur direkten Unterstützung eines Zahntechnikers und/oder Zahnarztes verstanden werden. Es sind verschiedene, einem Fachmann als sinnvoll erscheinende Dentalgeräte denkbar, insbesondere kann darunter beispielsweise eine Dentallaborabsaugung, wie insbesondere gemäß der EP 3 058 893 A1, eine Zahnarztabsaugung, ein dentales Kapselmischgerät, wie insbesondere gemäß der DE 10 2017 109 714 A1, ein dentales Strahlgerät, wie insbesondere gemäß der EP 3 015 085 A1, ein dentales Dampfstrahlgerät, eine Dentalbilderfassungsvorrichtung, wie insbesondere gemäß der EP 3 067 011 A1, eine dentale Arbeitsplatzleuchte, eine dentale CAD/CAM-Fräsmaschine oder dergleichen verstanden werden. Vorzugsweise ist das Dentalgerät in einer Laborumgebung und/oder einer Praxisumgebung mit mehreren Dentalgeräten angeordnet. Das Dentalgerät verfügt insbesondere über eine Steuer- und/oder Regeleinheit, welche zu einer Steuerung und/oder Regelung von Prozessen des Dentalgeräts vorgesehen ist. Die Steuer- und/oder Regeleinheit ist dabei insbesondere dazu vorgesehen, Prozesse des Dentalgeräts abhängig von Parametern anzupassen. Unter einer "Steuer- und/oder Regeleinheit" soll insbesondere eine Einheit mit zumindest einer Steuerelektronik verstanden werden. Unter einer "Steuerelektronik" soll insbesondere eine Einheit mit einer Prozessoreinheit und mit einer Speichereinheit sowie mit einem in der Speichereinheit gespeicherten Betriebsprogramm verstanden werden. Die Laborumgebung und/oder Praxisumgebung weist insbesondere eine gemeinsame Netzwerkumgebung auf und ist insbesondere in einem Gebäude angeordnet.

Unter einer "Kommunikationseinheit" soll in diesem Zusammenhang insbesondere eine Einheit verstanden werden, welche zu einer Bereitstellung einer, insbesondere kabellosen, Kommunikation, insbesondere einer Kommunikationsverbindung, mit dem Dentalgerät vorgesehen ist. Vorzugsweise weist die Kommunikationseinheit zu einer Kommunikation mit dem Dentalgerät zumindest eine Schnittstelle auf. Vorzugsweise soll unter einer Kommunikationseinheit insbesondere eine Einheit verstanden werden, welche zu einem Austausch von Daten vorgesehen ist. Insbesondere weist die Kommunikationseinheit zumindest einen Informationseingang und zumindest einen Informationsausgang auf. Vorzugsweise weist die Kommunikationseinheit zumindest zwei Informationseingänge und zumindest zwei Informationsausgänge auf, wobei jeweils zumindest ein Informationseingang und zumindest ein Informationsausgang zu einer Verbindung mit einem physischen System, insbesondere dem Bedienerendgerät und/oder einem Router und/oder einem Server, vorgesehen sind. Besonders bevorzugt soll darunter eine Schnittstelle zwischen zumindest zwei physischen Systemen, wie insbesondere zwischen dem Dentalgerät und dem Bedienerendgerät und/oder einem Router und/oder einem Server, verstanden werden. Es sind verschiedene, einem Fachmann als sinnvoll erscheinende Kommunikationseinheiten denkbar, insbesondere soll darunter jedoch eine drahtlose Schnittstelle, wie beispielsweise Bluetooth, WLAN, ZigBee, NFC, RFID, GSM, LTE oder UMTS, und/oder eine drahtgebundene Schnittstelle, wie beispielsweise ein USB-Anschluss, eine Canbus-Schnittstelle, eine RS485-Schnittstelle, eine SPI-Bus-Schnittstelle (Seriell Peripheral Interface), eine Ethernet-Schnittstelle, eine optische Schnittstelle, eine KNX-Schnittstelle und/oder eine Powerline-Schnittstelle, verstanden werden. Unter einer "Kommunikationsverbindung" soll insbesondere eine Verbindung verstanden werden, durch die eine Steuer- und/oder Regeleinheit mit weiteren Steuer- und/oder Regeleinheiten und/oder mit Sensoreinheiten verbunden ist oder wird, wodurch die Einheiten miteinander kommunizieren und Daten- und/oder Steuersignale austauschen können.

Ferner soll in diesem Zusammenhang unter einem "Bedienerendgerät" insbesondere ein Gerät zu einer direkten oder indirekten Kommunikation mit einem Bediener verstanden werden. Bevorzugt soll darunter insbesondere ein einem Bediener zugeordnetes Gerät verstanden werden. Vorzugsweise soll darunter insbesondere ein mobiles Gerät zur Kommunikation mit einem Bediener verstanden werden. Es sind verschiedene, einem Fachmann als sinnvoll erscheinende Bedienerendgeräte denkbar, insbesondere soll darunter jedoch ein Computer, ein Smartphone, ein Tablet-PC, ein Wearable Computer, insbesondere eine Smartwatch, und/oder eine Datenbrille, wie insbesondere eine AR-Brille und/oder ein Peripheral Head-Mounted Display (PHMD), verstanden werden. Als Bedienerendgerät wird insbesondere ein Smartphone oder Tablet-Computer favorisiert. Am Markt existiert eine Reihe von Smartphone- und Tablet-Systemen. Vorzugsweise umfasst das Bedienerendgerät insbesondere ein Sensormodul und/oder ein Kommunikationsmodul. Besonders bevorzugt ist das Bedienerendgerät insbesondere handhaltbar ausgebildet. Unter einem "Sensormodul" soll in diesem Zusammenhang insbesondere ein Modul mit zumindest einer Sensoreinheit, vorzugsweise mit einer Vielzahl verschiedener Sensoreinheiten, verstanden werden. Unter einem "Kommunikationsmodul" soll in diesem Zusammenhang insbesondere ein Modul verstanden werden, welches zumindest eine drahtlose und/oder kabelgebundene Kommunikationseinheit umfasst. Unter einer "Sensoreinheit" soll in diesem Zusammenhang insbesondere eine Einheit verstanden werden, die dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoreinheiten denkbar. Unter "dem Bediener zugeordnete Profilparameter" sollen in diesem Zusammenhang insbesondere Parameter eines Datenprofils verstanden werden, das insbesondere dem Bediener für das Dentalgerät zugeordnet ist. Vorzugsweise geben die Parameter Rückschluss auf das Datenprofil, welches zu einer Anpassung eines Betriebs des Dentalgeräts vorgesehen ist. Alternativ oder zusätzlich können die Profilparameter auch von dem Dentalgerät unabhängige bedienerspezifische Kenngrößen umfassen, wie beispielsweise Vitalwerte, allgemeine Kenntniswerte, Geräuschempfindlichkeit, Befugnisdaten oder dergleichen. In diesem Zusammenhang soll unter einem "Datenprofil" insbesondere ein Datensatz, bevorzugt ein digitaler Datensatz, verstanden werden, welcher ein oder mehrere Einstellungen und/oder Konfigurationen für das Dentalgerät umfasst. Vorzugsweise soll darunter insbesondere ein Datensatz verstanden werden, welcher zumindest eine Zuordnung aufweist. Bevorzugt ist der Datensatz einem Bediener und/oder einem Dentalgerät zugeordnet. Es ist insbesondere denkbar, dass die Datensätze zusätzlich von einem Bediener manuell, wie insbesondere an einem Computer in der Labor- und/oder Praxisumgebung manuell bearbeitet werden können.

Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung des Dentalsystems kann insbesondere eine schnelle Zuordnung von Einstellungen und/oder Konfigurationen erreicht werden. Insbesondere kann so eine komfortable Zuordnung erreicht werden. Vorzugsweise können so abhängig von einer aktuellen Situation und/oder einem aktuellen Bediener Profilparameter erfasst, insbesondere ausgewählt bzw. an- und/oder abgewählt, werden. So kann insbesondere eine schnelle Einstellung erreicht werden. Hierdurch kann wiederum ein vorteilhaft hoher Komfort erreicht werden.

Ferner wird vorgeschlagen, dass das Bedienerendgerät zu einer Anpassung zumindest eines Prozessparameters des Dentalgeräts abhängig von dem zumindest einen Profilparameter vorgesehen ist. Die Prozessparameter und die Profilparameter sind insbesondere demselben Datenprofil zugeordnet. Die Prozessparameter umfassen insbesondere ein oder mehrere Einstellungen und/oder Konfigurationen für das Dentalgerät. Vorzugsweise sind die Prozessparameter mit den Profilparametern gekoppelt. Insbesondere werden die Prozessparameter von den Profilparametern abgeleitet. Abhängig von den ermittelten Profilparametern werden insbesondere die Prozessparameter bestimmt und entsprechend in dem Dentalgerät eingestellt. Unter "Prozessparametern" sollen in diesem Zusammenhang insbesondere einen Betrieb des Dentalgeräts beeinflussende Parameter verstanden werden. Vorzugsweise sollen darunter insbesondere variable Parameter des Dentalgeräts verstanden werden, welche abhängig von einem Betriebszustand variabel von der Steuer- und/oder Regeleinheit einstellbar sind. Dadurch kann insbesondere eine selbsttätige Prozessanpassung des Dentalgeräts erreicht werden. Ein Prozess, insbesondere Prozessparameter, des Dentalgeräts kann insbesondere anhand von dem Bediener zugeordneten Profilparametern angepasst werden.

Des Weiteren wird vorgeschlagen, dass das Bedienerendgerät dazu vorgesehen ist, abhängig von einem erfassten Bediener, zumindest einen Energieparameter des Dentalgeräts über die Kommunikationseinheit einzustellen. Vorzugsweise ist das Bedienerendgerät insbesondere dazu vorgesehen ist, abhängig von einem erfassten Bediener, insbesondere abhängig von einem Nutzerverhalten, ein Energiemanagement des Dentalgeräts über die Kommunikationseinheit anzupassen. Bei dem Nutzerverhalten werden insbesondere wiederkehrende Prozessabläufe erfasst. Bevorzugt kann mittels des Bedienerendgeräts insbesondere zumindest ein ECO-Modus des Dentalgeräts über die Kommunikationseinheit gesteuert, insbesondere aktiviert und/oder deaktiviert, werden und/oder es können einem Bediener ECO-Empfehlungen ausgegeben werden, die einen Bediener bei einer energieeffizienten Nutzung des Dentalgeräts unterstützen. Unter einem "Energieparameter des Dentalgeräts" soll in diesem Zusammenhang insbesondere ein einen Energieverbrauch des Dentalgeräts, insbesondere wesentlich, beeinflussender Parameter verstanden werden, wie beispielsweise ein Leistungsparameter und/oder ein Regelungsparameter. Insbesondere kann in einem ECO-Modus beispielsweise mittels der Energieparameter eine Ausgabeleistung reduziert, ein Regelverhalten verlangsamt und/oder eine Stand-by-Verhalten verändert werden. Es ist jedoch auch denkbar, dass der Energieparameter insbesondere lediglich indirekt, insbesondere abhängig von einem Nutzerverhalten, einen Energieverbrauch des Dentalgeräts beeinflusst. Dadurch kann insbesondere eine vorteilhaft energieeffiziente Nutzung des Dentalgeräts erreicht werden. Es kann insbesondere automatisch ein Energieparameter des Dentalgeräts eingestellt werden. Insbesondere kann ein Energieparameter des Dentalgeräts an einen Bediener angepasst werden.

Es wird ferner vorgeschlagen, dass das Bedienerendgerät dazu vorgesehen ist, den Energieparameter abhängig von einem mittels des Dentalgeräts erfassten und mittels des Bedienerendgeräts gespeicherten Nutzerverhaltens des Bedieners festzulegen. Vorzugsweise ist das Bedienerendgerät dazu vorgesehen, ein Nutzerverhalten des Bedieners zu dokumentieren und auszuwerten. Bevorzugt verfügt das Bedienerendgerät über einen Auswertealgorithmus und/oder eine künstliche Intelligenz, welche/r abhängig von dem erfassten Nutzerverhalten optimierte Energieparameter des Dentalgeräts ermittelt und insbesondere auf das Dentalgerät überträgt. Hierdurch kann insbesondere ein Energiemanagement des Dentalgeräts abhängig von einem Nutzerverhalten angepasst werden. Eine Anpassung kann dabei insbesondere kontinuierlich erfolgen, wobei die Energieparameter ständig abhängig von einem Nutzerverhalten angepasst werden. Dadurch kann insbesondere eine vorteilhafte Anpassung eines Energiemanagements des Dentalgeräts erreicht werden. Es kann insbesondere eine vorteilhaft angepasste Einstellung eines Energieparameters erreicht werden.

Es wird weiter vorgeschlagen, dass das Bedienerendgerät dazu vorgesehen ist, mittels des Dentalgeräts erfasste Nutzungsdaten eines Bedieners auszuwerten und dem Bediener Handlungsempfehlungen, insbesondere zu einer Prozessoptimierung, auszugeben. Vorzugsweise ist das Bedienerendgerät dazu vorgesehen, ein Nutzerverhalten des Bedieners zu dokumentieren und auszuwerten. Bevorzugt verfügt das Bedienerendgerät über einen Auswertealgorithmus und/oder eine künstliche Intelligenz, welche/r abhängig von dem erfassten Nutzerverhalten optimierte Prozessvorgänge und/oder Prozessparameter des Dentalgeräts ermittelt. Insbesondere werden dem Bediener die optimierten Prozessvorgänge in Form von Handlungsempfehlungen ausgegeben. Die Handlungsempfehlungen können dabei insbesondere das Anpassen von Abläufen, die Verwendung von Produkten oder dergleichen beinhalten. Insbesondere ist denkbar, dass die Handlungsempfehlungen und/oder die Erfassung der Nutzungsdaten eines Bedieners geräteübergreifend, also insbesondere bei mehreren Dentalgeräten einer Laborumgebung und/oder einer Praxisumgebung, erfolgen. Vorzugsweise können die Handlungsempfehlungen dadurch insbesondere auch geräteübergreifende Prozesse betreffen. Dadurch kann insbesondere eine vorteilhaft komfortable und angepasste Nutzung des Dentalgeräts erreicht werden. Es kann ferner insbesondere eine Arbeitseffizienz und/oder eine Energieeffizienz an dem zumindest einen Dentalgerät verbessert werden. Es kann insbesondere direkt eine Einsparmöglichkeit angeboten und vorzugsweise verkauft werden.

Ferner wird vorgeschlagen, dass das Dentalsystem zumindest ein weiteres Dentalgerät aufweist, welches zumindest eine Steuer- und/oder Regeleinheit aufweist, die zu einer Steuerung und/oder Regelung zumindest eines Prozesses des weiteren Dentalgeräts vorgesehen ist, mit zumindest einer mit dem weiteren Dentalgerät gekoppelten weiteren Kommunikationseinheit, wobei das Bedienerendgerät dazu vorgesehen ist, über die Kommunikationseinheit das Dentalgerät abhängig von zumindest einem Parameter des weiteren Dentalgeräts anzusteuern. Vorzugsweise ist das Bedienerendgerät dazu vorgesehen, die Parameter des weiteren Dentalgeräts über die weitere Kommunikationseinheit zu empfangen. Bevorzugt werden durch die Kopplung des Dentalgeräts und des weiteren Dentalgeräts mit dem Bedienerendgerät Synergiefunktionen zwischen den Dentalgeräten erzeugt. Das Bedienerendgerät ist insbesondere zu einer voneinander abhängigen Ansteuerung der Dentalgeräte vorgesehen. Insbesondere ist das Bedienerendgerät dazu vorgesehen, abhängig von einem Betriebszustand des weiteren Dentalgeräts das Dentalgerät anzusteuern. Es wäre insbesondere auch denkbar, das Bedienerendgerät das Dentalgerät abhängig von einem Vorhandensein des weiteren Dentalgeräts in einer Laborumgebung und/oder einer Praxisumgebung anzusteuern. Dadurch kann insbesondere vorteilhaft über das Bedienerendgerät eine Synergie zwischen verschiedenen Dentalgeräten erzeugt werden. Intelligente Funktionen zwischen den Dentalgeräten können insbesondere über das Bedienerendgerät bereitgestellt und gesteuert werden. Es kann insbesondere eine schnelle und insbesondere automatische Konfiguration einer Laborumgebung und/oder einer Praxisumgebung erreicht werden. So kann insbesondere eine schnelle Einstellung erreicht werden. Hierdurch kann wiederum ein vorteilhaft hoher Komfort erreicht werden.

Des Weiteren wird vorgeschlagen, dass das Bedienerendgerät über die Kommunikationseinheit und die weitere Kommunikationseinheit zu einer gleichzeitigen Ansteuerung des Dentalgeräts und des weiteren Dentalgeräts vorgesehen ist. Vorzugsweise ist das Bedienerendgerät dazu vorgesehen, die Parameter des weiteren Dentalgeräts über die weitere Kommunikationseinheit zu empfangen. Bevorzugt werden durch die Kopplung des Dentalgeräts und des weiteren Dentalgeräts mit dem Bedienerendgerät Synergiefunktionen zwischen den Dentalgeräten erzeugt. Das Bedienerendgerät ist insbesondere zu einer voneinander abhängigen Ansteuerung der Dentalgeräte vorgesehen. Es kann mittels des Bedienerendgeräts insbesondere eine gleichzeitige Steuerung mehrerer Dentalgeräte in einer Laborumgebung und/oder einer Praxisumgebung erreicht werden. Das Dentalgerät und das weitere Dentalgerät sind insbesondere von verschiedenen Geräten gebildet, welche jedoch in einem funktionalen Zusammenhang stehen. Dadurch kann insbesondere vorteilhaft über das Bedienerendgerät eine Synergie zwischen verschiedenen Dentalgeräten erzeugt werden.

Es kann insbesondere eine schnelle und insbesondere automatische Konfiguration einer Laborumgebung und/oder einer Praxisumgebung erreicht werden.

Es wird ferner vorgeschlagen, dass das Bedienerendgerät dazu vorgesehen ist, ein mittels des zumindest einen Dentalgeräts erfasstes Nutzerverhalten auszuwerten und auf einer Speichereinheit zu hinterlegen. Unter einer "Speichereinheit" soll insbesondere eine Einheit verstanden werden, die dazu vorgesehen ist, zumindest eine Information, vorteilhaft von einer Stromversorgung unabhängig, zu speichern. Die Speichereinheit kann insbesondere als ein Teil des Bedienerendgeräts ausgebildet sein sowie von einer externen Speichereinheit, wie insbesondere einem Server, gebildet sein. Vorzugsweise weist das Bedienerendgerät die Speichereinheit auf. Die Speichereinheit ist insbesondere von einem internen Speicher des Bedienerendgeräts gebildet. Dadurch kann insbesondere eine vorteilhafte Dokumentation des Nutzerverhaltens erreicht werden. Ferner kann dadurch insbesondere eine dauerhafte und/oder rückwirkende Auswertung eines Nutzerverhaltens erreicht werden.

Es wird weiter vorgeschlagen, dass das Dentalsystem eine Servereinheit aufweist, wobei das Bedienerendgerät dazu vorgesehen ist, ein gespeichertes Nutzerverhalten auf die Servereinheit zu übertragen. Vorzugsweise ist das Bedienerendgerät mittels einer Internetverbindung, insbesondere mittels einer Mobilfunkverbindung, mit der Servereinheit verbunden. Vorzugsweise werden die Nutzerdaten auf einer zentralen Servereinheit gespeichert und können so unabhängig von einem Standort abgerufen werden. Die Servereinheit ist insbesondere von einem Server und/oder einer Cloud gebildet. Es wäre auch denkbar, dass die Servereinheit von einem lokalen Server der Laborumgebung und/oder einer Praxisumgebung gebildet ist. Dadurch kann insbesondere eine vorteilhafte Dokumentation des Nutzerverhaltens erreicht werden. Ferner kann dadurch insbesondere eine dauerhafte und/oder rückwirkende Auswertung eines Nutzerverhaltens erreicht werden. Es kann zudem insbesondere eine bedienerübergreifende Auswertung eines Nutzerverhaltens auf der Servereinheit erfolgen.

Ferner wird vorgeschlagen, dass die Servereinheit dazu vorgesehen ist, insbesondere bedienerübergreifend, Daten eines Nutzerverhaltens auszuwerten und daraus optimierte Prozessparameter abzuleiten. Vorzugsweise werden die Nutzdaten auf einer zentralen Servereinheit gesammelt und ausgewertet. Hierdurch können insbesondere optimierte Prozessparameter und/oder Prozessabläufe abgeleitet werden. Die Erfassung des Nutzerverhaltens erfolgt dabei insbesondere bedienerübergreifend und laborumgebungsübergreifend. Die Servereinheit ist insbesondere dazu vorgesehen, die Daten eines Nutzerverhaltens laborumgebungsübergreifend auszuwerten und daraus für alle Bediener optimierte Prozessparameter und/oder Handlungsempfehlungen abzuleiten. Es wäre insbesondere denkbar, dass ein Nutzerverhalten eines Bedieners mit vorteilhaften Prozessparametern und/oder Prozessabläufen dazu genutzt wird, anderen Nutzern Handlungsempfehlungen oder verbesserte Prozessparameter auszugeben, wobei dies insbesondere laborumgebungsübergreifend erfolgen kann. Die Erfahrungswerte von tausenden Nutzern eines Dentalgeräts können so insbesondere einem Bediener zu Gute kommen. Es wäre insbesondere denkbar, dass so jeder Bediener von anderen Bedienern lernen kann. Hierdurch kann insbesondere eine bedienerübergreifende, insbesondere globale, Optimierung von Prozessparametern und/oder Prozessabläufen erreicht werden. Es wäre insbesondere denkbar, dass die Optimierung von Prozessparametern und/oder Prozessabläufen und/oder Handlungsempfehlungen mit einem Bezahldienst gekoppelt sind, sodass für die Optimierung von Prozessparametern und/oder Prozessabläufen und/oder für Handlungsempfehlungen bezahlt werden kann und/oder muss. Es kann insbesondere ein Geschäftsmodell bereitgestellt werden.

Des Weiteren wird vorgeschlagen, dass das Bedienerendgerät dazu vorgesehen ist, über die Kommunikationseinheit einen Wartungszugriff, insbesondere einen Wartungsfernzugriff, auf das Dentalgerät bereitzustellen. Insbesondere kann über das Bedienerendgerät ein Wartungszugriff auf das Dentalgerät über das Internet hergestellt werden. Hierdurch kann insbesondere ein Fernzugriff zu Wartungszwecken ermöglicht werden. Der Wartungszugriff dient insbesondere für einen Servicemitarbeiter zu einer Wartung des Dentalgeräts und/oder zu einem externen Aufspielen von Updates. Vorzugsweise kann über den Wartungszugriff von einer externen Servicestelle aus auf die Steuer- und/oder Regeleinheit des Dentalgeräts zugegriffen werden. Bevorzugt können über den Wartungszugriff von einer externen Servicestelle aus Parameter des Dentalgeräts angepasst werden. Vorzugsweise kann über den Wartungszugriff insbesondere auf ein Fehlerprotokoll oder dergleichen des Dentalgeräts zugegriffen werden. Dadurch kann insbesondere eine Wartung des Dentalgeräts ermöglicht werden, ohne dass ein Servicemitarbeiter vor Ort sein muss. Es kann insbesondere eine vorteilhaft komfortable Wartung ermöglicht werden.

Ferner geht die Erfindung aus von einer Kommunikationseinheit des Dentalsystems.

Des Weiteren geht die Erfindung aus von einem Verfahren zu einem Betrieb des Dentalsystems. Es wird vorgeschlagen, dass bei dem Verfahren in zumindest einem, insbesondere automatischen, Anmeldeschritt das Bedienerendgerät dem Bediener zugeordnete Profilparameter für das Dentalgerät abruft. Vorzugsweise werden mittels des Bedienerendgeräts dem Bediener zugeordnete Profilparameter abgerufen und insbesondere davon abhängige Prozessparameter auf das Dentalgerät übertragen. Die dem Bediener zugeordneten Profilparameter können dabei insbesondere sowohl direkt auf dem Bedienerendgerät hinterlegt sein als auch auf einer Datenbank eines externen oder internen Servers. Dadurch kann insbesondere eine schnelle Zuordnung von Einstellungen und/oder Konfigurationen erreicht werden. Insbesondere kann so eine komfortable Zuordnung erreicht werden. Vorzugsweise können so abhängig von einer aktuellen Situation und/oder einem aktuellen Bediener Profilparameter erfasst, insbesondere ausgewählt bzw. an- und/oder abgewählt, werden. So kann insbesondere eine schnelle Einstellung erreicht werden. Hierdurch kann wiederum ein vorteilhaft hoher Komfort erreicht werden.

Es wird ferner vorgeschlagen, dass in dem zumindest einen Anmeldeschritt Prozessparameter des Dentalgeräts abhängig von dem zumindest einen, dem Bediener zugeordneten Profilparameter angepasst werden. Die Prozessparameter und die Profilparameter sind insbesondere demselben Datenprofil zugeordnet. Abhängig von den ermittelten Profilparametern werden insbesondere die Prozessparameter bestimmt und entsprechend in dem Dentalgerät eingestellt. Dadurch kann insbesondere eine selbsttätige Prozessanpassung des Dentalgeräts erreicht werden. Ein Prozess, insbesondere Prozessparameter, des Dentalgeräts kann insbesondere anhand von dem Bediener zugeordneten Profilparametern angepasst werden.

Es wird weiter vorgeschlagen, dass in zumindest einem Verfahrensschritt, insbesondere mittels des Bedienerendgeräts, abhängig von einem erfassten Bediener zumindest eine Gerätefunktion des Dentalgeräts freigegeben und/oder gesperrt wird. Vorzugsweise können den verschiedenen Bedienern verschiedene Berechtigungen zugewiesen werden, welche wiederum unterschiedliche Gerätefunktion des Dentalgeräts freigeben und/oder sperren. Bevorzugt sind insbesondere verschiedene Benutzergruppen vorgesehen, in welche die Bediener einsortiert werden können, wobei abhängig von der Benutzergruppe verschiedene Gerätefunktion des Dentalgeräts freigegeben und/oder gesperrt sind. Die Gerätefunktionen können dabei insbesondere abhängig von einer Qualifikation des Bedieners und/oder der Benutzergruppe freigegeben werden. So können beispielsweise bei einem dentalen Strahlgerät für einen Zahntechniker und/oder Zahnarzt höher Drücke oder dergleichen freigegeben werden. Dadurch kann insbesondere eine vorteilhaft angepasste Funktionalität des zumindest einen Dentalgeräts erreicht werden. Es kann insbesondere ein vorteilhaft einfaches und komfortables Freigabesystem bereitgestellt werden.

Zudem wird vorgeschlagen, dass in zumindest einem Verfahrensschritt das Bedienerendgerät über die Kommunikationseinheit das Dentalgerät abhängig von einem Status und/oder einem Betriebszustand eines weiteren Dentalgeräts, insbesondere eines weiteren Dentalgeräts derselben Laborumgebung, ansteuert. Vorzugsweise wird der Status und/oder der Betriebszustand des weiteren Dentalgeräts über die weitere Kommunikationseinheit empfangen. Bevorzugt werden durch die Kopplung des Dentalgeräts und des weiteren Dentalgeräts mit dem Bedienerendgerät Synergiefunktionen zwischen den Dentalgeräten erzeugt. Das Bedienerendgerät steuert die Dentalgeräte insbesondere voneinander abhängig an. Insbesondere steuert das Bedienerendgerät abhängig von einem Betriebszustand des weiteren Dentalgeräts das Dentalgerät an. Es wäre insbesondere denkbar, das Bedienerendgerät das Dentalgerät abhängig von einem Aktivierungszustand des weiteren Dentalgeräts in einer Laborumgebung und/oder einer Praxisumgebung anzusteuern. Dadurch kann insbesondere vorteilhaft über das Bedienerendgerät eine Synergie zwischen verschiedenen Dentalgeräten erzeugt werden. Es kann insbesondere eine schnelle und insbesondere automatische Konfiguration einer Laborumgebung und/oder einer Praxisumgebung erreicht werden. So kann insbesondere eine schnelle Einstellung erreicht werden. Hierdurch kann wiederum ein vorteilhaft hoher Komfort erreicht werden.

Ferner wird vorgeschlagen, dass in zumindest einem Optimierungsschritt dem Bediener über das Bedienerendgerät abhängig von einem mittels des Dentalgeräts erfassten Nutzerverhalten Prozessoptimierungen angeboten werden. Vorzugsweise dokumentiert das Bedienerendgerät ein Nutzerverhalten des Bedieners und wertet dieses aus. Bevorzugt verfügt das Bedienerendgerät über einen Auswertealgorithmus und/oder eine künstliche Intelligenz, welche/r abhängig von dem erfassten Nutzerverhalten optimierte Prozessvorgänge und/oder Prozessparameter des Dentalgeräts ermittelt. Insbesondere werden dem Bediener die optimierten Prozessvorgänge in Form von Handlungsempfehlungen ausgegeben. Die Handlungsempfehlungen können dabei insbesondere das Anpassen von Abläufen, die Verwendung von Produkten oder dergleichen beinhalten. Insbesondere ist denkbar, dass die Handlungsempfehlungen und/oder die Erfassung der Nutzungsdaten eines Bedieners geräteübergreifend, also insbesondere bei mehreren Dentalgeräten einer Laborumgebung und/oder einer Praxisumgebung, erfolgen. Vorzugsweise können die Handlungsempfehlungen dadurch insbesondere auch geräteübergreifende Prozesse betreffen. Dadurch kann insbesondere eine vorteilhaft komfortable und angepasste Nutzung des Dentalgeräts erreicht werden. Es kann ferner insbesondere eine Arbeitseffizienz und/oder eine Energieeffizienz an dem zumindest einen Dentalgerät verbessert werden.

Des Weiteren wird vorgeschlagen, dass in zumindest einem Anpassungsschritt mittels des Bedienerendgeräts Produktinformationen abgerufen werden und Prozessparameter des Dentalgeräts abhängig von abgerufenen Produktinformationen angepasst werden. Unter "Produktinformationen" sollen in diesem Zusammenhang insbesondere Informationen, insbesondere technische Informationen, zu einem Produkt verstanden werden, welches zu einer Verwendung mit dem zumindest einen Dentalgerät vorgesehen ist. Die Produktinformationen können beispielsweise Mischparameter, wie Mischungsverhältnisse, Mischzeiten oder dergleichen, Viskositäten, Abbindezeiten, Aushärtungsparameter oder dergleichen umfassen. Es sind verschiedene, einem Fachmann als sinnvoll erscheinende Produktinformationen denkbar. Die Produktinformationen stammen insbesondere von einem Produkthersteller. Beispielsweise wäre denkbar, dass bei einem als dentales Kapselmischgerät und/oder dentales Anmischgerät ausgebildetes Dentalgerät selbsttätig Produktinformationen zu einer aktuell verwendeten Kapsel und/oder einer aktuell verwendeten Kartusche automatisch abgerufen werden und Prozessparameter des Dentalgeräts abhängig von den abgerufenen Produktinformationen angepasst werden. Eine Erkennung der aktuell verwendeten Kapsel und/oder der aktuell verwendeten Kartusche kann beispielsweise automatisiert in dem Dentalgerät erfolgen oder beispielsweise durch Scannen mittels des Bedienerendgeräts.

Das erfindungsgemäße Dentalsystem, die Kommunikationseinheit sowie das Verfahren sollen hierbei nicht auf die oben beschriebene Anwendung und Ausführungsform beschränkt sein. Insbesondere können das erfindungsgemäße Dentalsystem, die Kommunikationseinheit sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein Dentalsystem mit mehreren Dentalgeräten, mit mehreren universellen Kommunikationsvorrichtungen und mit einem Bedienerendgerät in einer schematischen Darstellung,
- Fig. 2: eine Laborumgebung mit dem Dentalsystem und einen Bediener in einer schematischen Darstellung und
- Fig. 3: ein schematisches Ablaufdiagramm eines Verfahrens zu einem Betrieb des Dentalsystems.

### Beschreibung des Ausführungsbeispiels

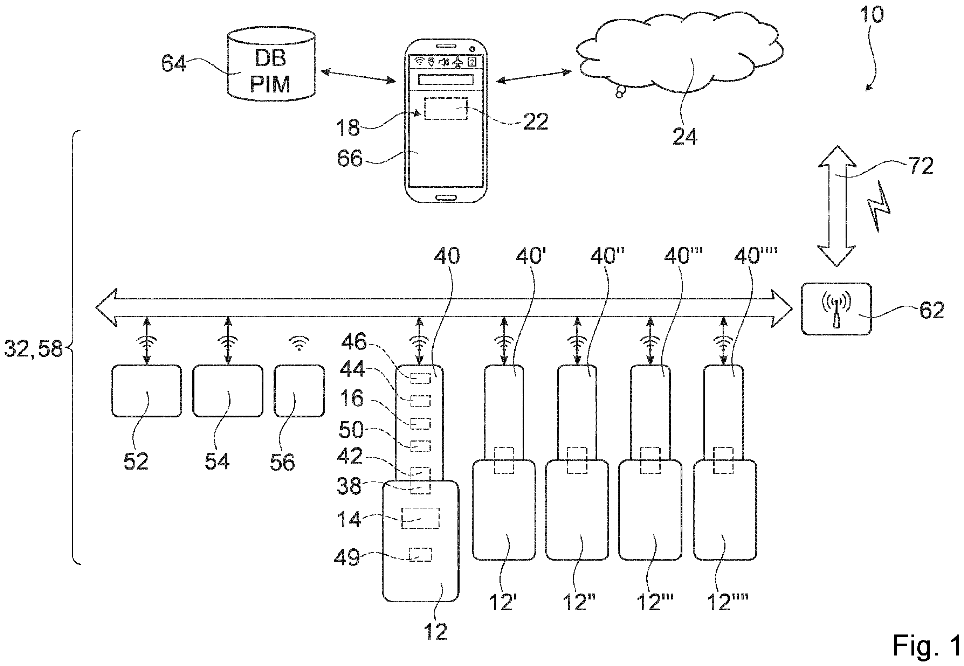

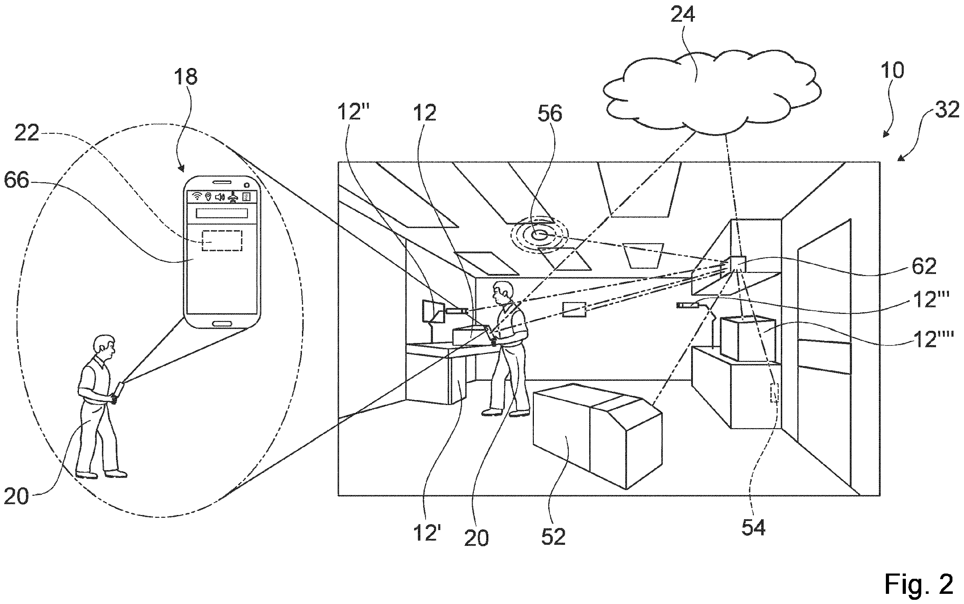

Die Figuren 1 und 2 zeigen ein Dentalsystem 10. Das Dentalsystem 10 befindet sich in einer Laborumgebung 32 eines Dentallabors. Es wäre jedoch auch denkbar, dass sich das Dentalsystem 10 in einer Praxisumgebung eines Zahnarztes befindet. Das Dentalsystem 10 umfasst mehrere Dentalgeräte 12, 12', 12", 12"', 12"". Die Dentalgeräte 12, 12', 12", 12'", 12"" befinden sich in der Laborumgebung 32 und sind beispielsweise in einem Laborraum angeordnet. Ein erstes Dentalgerät 12 ist von einem Kapselmischgerät, wie insbesondere gemäß der DE 10 2017 109 714 A1, gebildet. Ein zweites Dentalgerät 12' ist von einer Dentallaborabsaugung, wie insbesondere gemäß der EP 3 058 893 A1, gebildet. Ein drittes Dentalgerät 12" ist von einer Dentalbilderfassungsvorrichtung, wie insbesondere gemäß der EP 3 067 011 A1, gebildet. Ein viertes Dentalgerät 12'" ist von einer Arbeitsplatzleuchte gebildet. Ein fünftes Dentalgerät 12"" ist von einem dentalen Dampfstrahlgerät gebildet. Grundsätzlich wäre jedoch auch eine andere, einem Fachmann als sinnvoll erscheinende Ausbildung der Dentalgeräte 12, 12', 12", 12"', 12"" denkbar. Die Ausbildung der Dentalgeräte 12, 12', 12", 12'", 12"" ist insbesondere von einem Aufbau des Labors und/oder der Praxis abhängig.

Die Dentalgeräte 12, 12', 12", 12"', 12"" weisen jeweils eine Steuer- und/oder Regeleinheit 14 auf, wobei in der Figur 1 lediglich die Steuer- und/oder Regeleinheit 14 des ersten Dentalgeräts 12 schematisch dargestellt ist. Die Steuer- und/oder Regeleinheiten 14 der Dentalgeräte 12, 12', 12", 12"', 12"" sind jeweils zu einer Steuerung und/oder Regelung der Prozesse des jeweiligen Dentalgeräts 12, 12', 12", 12"', 12"" vorgesehen.

Ferner weisen die Dentalgeräte 12, 12', 12", 12'", 12"" jeweils eine Datenschnittstelle 38 auf, wobei in der Figur 1 lediglich die Datenschnittstelle 38 des ersten Dentalgeräts 12 schematisch dargestellt ist. Die Datenschnittstellen 38 sind jeweils direkt mit einer der Steuer- und/oder Regeleinheiten 14 gekoppelt. Die Datenschnittstellen 38 sind jeweils von einer SPI-Bus-Schnittstelle (Seriell Peripheral Interface)gebildet. Es wäre jedoch auch denkbar, dass die Datenschnittstellen 38 von USB-Schnittstellen gebildet sind.

Ferner weisen die Dentalgeräte 12, 12', 12", 12"', 12"" jeweils Sensoren 48 auf, wobei in der Figur 1 lediglich ein Sensor 48 des ersten Dentalgeräts 12 schematisch dargestellt ist. Die Sensoren 48 können dabei je nach Ausgestaltung des Dentalgeräts 12, 12', 12", 12"', 12"" verschiedene Ausgestaltungen haben. Die Dentalgeräte 12, 12', 12", 12"', 12"" können beispielsweise Drucksensoren, optische Sensoren, Beschleunigungssensoren, Strömungsmesser oder dergleichen umfassen. Der Sensor 48 des ersten Dentalgeräts 12 ist beispielhaft von einem Beschleunigungssensor zu einer Erfassung einer Beschleunigung eines Schwingarms zu einer Aufnahme einer Mischkapsel gebildet.

Des Weiteren weist das Dentalsystem 10 mehrere universelle Kommunikationsvorrichtungen 40, 40', 40", 40"', 40"" auf. Die universellen Kommunikationsvorrichtungen 40, 40', 40", 40"', 40"" sind jeweils identisch ausgebildet. Die Beschreibung der ersten Kommunikationsvorrichtung 40 kann daher grundsätzlich auch auf die weiteren Kommunikationsvorrichtungen 40', 40", 40"', 40"" angewendet werden. Die universellen Kommunikationsvorrichtungen 40, 40', 40", 40"', 40"" sind jeweils mit verschiedenen Dentalgeräten 12, 12', 12", 12'", 12"" koppelbar, welche jeweils die gleiche Datenschnittstelle 38 aufweisen. Die Dentalgeräte 12, 12', 12", 12"', 12"" verfügen jeweils über eine standardisierte Datenschnittstelle 38. Die universellen Kommunikationsvorrichtungen 40, 40', 40", 40"', 40"" sind jeweils variabel von dem jeweiligen Dentalgerät 12, 12', 12", 12"', 12"" trennbar ausgebildet. Die universellen Kommunikationsvorrichtungen 40, 40', 40", 40"', 40"" können jeweils werkzeuglos, von dem jeweiligen Dentalgerät 12, 12', 12", 12"', 12"" getrennt werden. Die universellen Kommunikationsvorrichtungen 40, 40', 40", 40"', 40"" sind mittels einer lösbaren Steckverbindung mit dem jeweiligen Dentalgerät 12, 12', 12", 12"', 12"" koppelbar. Die universellen Kommunikationsvorrichtungen 40, 40', 40", 40"', 40"" weisen dazu jeweils eine direkt, mechanisch mit der Datenschnittstelle 38 der Dentalgeräte 12, 12', 12", 12"', 12"" koppelbare Datenschnittstelle 42 auf. In der Figur 1 ist der Übersichtlichkeit halber lediglich die Datenschnittstelle 42 der ersten universellen Kommunikationsvorrichtung 40 dargestellt. Die Datenschnittstellen 42 sind jeweils von SPI-Bus-Schnittstellen (Seriell Peripheral Interface)gebildet. Es wäre jedoch auch denkbar, dass die Datenschnittstellen 42 von USB-Schnittstellen gebildet sind. Die universellen Kommunikationsvorrichtungen 40, 40', 40", 40'", 40"" sind jeweils von einem Datenstick gebildet. Die universellen Kommunikationsvorrichtungen 40, 40', 40", 40"', 40"" sind jeweils von einem WLAN-Datenstick gebildet. Die universellen Kommunikationsvorrichtungen 40, 40', 40", 40"', 40"" können zu einer Vernetzung der Dentalgeräte 12, 12', 12", 12"', 12"" jeweils optional in die Dentalgeräte 12, 12', 12", 12"', 12"" eingesteckt werden. Die universellen Kommunikationsvorrichtungen 40, 40', 40", 40"', 40"" können variabel mit den Dentalgeräten 12, 12', 12", 12"', 12"" verbunden oder von diesen getrennt werden. Die Dentalgeräte 12, 12', 12", 12"', 12"" können mittels der Kommunikationsvorrichtungen 40, 40', 40", 40"', 40"" erweitert werden. Die Dentalgeräte 12, 12', 12", 12"', 12"" nutzen die universellen Kommunikationsvorrichtungen 40, 40', 40", 40"', 40"" jeweils als Kommunikationsgerät. Die universellen Kommunikationsvorrichtungen 40, 40', 40", 40"', 40"" weisen jeweils eine Kommunikationseinheit 16 auf. In der Figur 1 ist der Übersichtlichkeit halber lediglich die Kommunikationseinheit 16 der ersten universellen Kommunikationsvorrichtung 40 dargestellt. Die Kommunikationseinheiten 16 sind zu einer drahtlosen Kommunikation mit einer Servereinheit 24 und/oder einem Bedienerendgerät 18 vorgesehen. Die Kommunikationseinheiten 16 sind dazu vorgesehen, sich mit einem Netzwerk der Laborumgebung 32 zu verbinden und sich über das Netzwerk mit einer Servereinheit 24 und/oder einem Bedienerendgerät 18 zu verbinden. Die Kommunikationseinheiten 16 sind mit jeweils einem der Dentalgeräte 12, 12', 12", 12"', 12"" gekoppelt. Die Kommunikationseinheiten 16 sind jeweils über die Datenschnittstellen 38, 42 mit einem der Dentalgeräte 12, 12', 12", 12'", 12"" gekoppelt.

Ferner weisen die universellen Kommunikationsvorrichtungen 40, 40', 40", 40"', 40"" jeweils eine Recheneinheit 44 auf. In der Figur 1 ist der Übersichtlichkeit halber lediglich die Recheneinheit 44 der ersten universellen Kommunikationsvorrichtung 40 dargestellt. Die Recheneinheit 44 ist dazu vorgesehen, zumindest einen Rechenprozess des Dentalgeräts 12, 12', 12", 12'", 12"", mit welchem die jeweilige der universellen Kommunikationsvorrichtung 40, 40', 40", 40"', 40"" gekoppelt ist, auszuführen. Das entsprechende Dentalgerät 12, 12', 12", 12"', 12"" kann dazu komplexe, insbesondere rechenaufwendige, Verarbeitungsprozesse auf die Recheneinheit 44 der gekoppelten Kommunikationsvorrichtung 40, 40', 40", 40"', 40"" auslagern, insbesondere um eine eigene Rechenleistung zu reduzieren und für prozessnotwenige Rechenaufgaben zu nutzen. So können insbesondere komplexe Berechnungen und/oder Algorithmen auf der Recheneinheit 44 der Kommunikationsvorrichtung 40, 40', 40", 40"', 40"" ausgeführt werden. Die komplexen Berechnungen und Algorithmen werden insbesondere lediglich ausgeführt, wenn eine der universellen Kommunikationsvorrichtungen 40, 40', 40", 40"', 40"" an das Dentalgerät 12, 12', 12", 12"', 12"" angeschlossen ist. Ist keine universelle Kommunikationsvorrichtung 40, 40', 40", 40"', 40"" an das Dentalgerät 12, 12', 12", 12"', 12"" angeschlossen, wird auf eine entsprechende Berechnung verzichtet.

Bei dem dritten Dentalgerät 12" kann beispielsweise eine komplexe Berechnung einer Bildkorrektur zu einer optimalen Darstellung einer Zahnprothese und/oder eine Bildbearbeitung und/oder ein Bildhandling auf der Recheneinheit der dritten universellen Kommunikationsvorrichtung 40" berechnet werden. Alternativ wäre auch denkbar, dass entsprechende Berechnungen auf der Servereinheit 24 oder dem Bedienerendgerät 18 durchgeführt werden. Das dritte Dentalgerät 12" kann dadurch insbesondere auf eine optimale Performance ausgelegt werden. Ferner können über das Bedienerendgerät 18 insbesondere vorzugsweise Bilder oder Momentaufnahmen von dem dritten Dentalgerät 12" heruntergeladen und/oder auf das dritte Dentalgerät 12" geladen werden. Hierdurch kann insbesondere vorteilhaft ein Split-Screen-Modus realisiert werden, in welchem zusätzliche Bilder eingeblendet werden können. Ferner kann an dem Bedienerendgerät 18 eine Bildbearbeitung, wie insbesondere eine Bildnachbearbeitung, das Erstellen von Teilbildern, das Einblenden von Hilfslinien oder dergleichen realisiert werden. Für die Bildbearbeitung können über das Bedienerendgerät 18 insbesondere vorteilhaft Applikationen von Drittanbietern genutzt werden.

Des Weiteren weisen die universellen Kommunikationsvorrichtungen 40, 40', 40", 40"', 40"" jeweils eine Sensoreinheit 46 zu einer Erfassung zumindest eines Umgebungsparameters auf. In der Figur 1 ist der Übersichtlichkeit halber lediglich die Sensoreinheit 46 der ersten universellen Kommunikationsvorrichtung 40 dargestellt. Die Sensoreinheit 46 ist beispielhaft zu einer Erfassung einer Umgebungstemperatur und einer Umgebungsfeuchtigkeit vorgesehen. Es wäre jedoch auch denkbar, dass die Sensoreinheit 46 zu einer Erfassung anderer Umgebungsparameter vorgesehen ist. Die universellen Kommunikationsvorrichtungen 40, 40', 40", 40"', 40"" sind dazu vorgesehen, Prozessparameter des jeweils zugeordneten Dentalgeräts 12, 12', 12", 12'", 12"" abhängig von einem erfassten Umgebungsparameter anzupassen. Die universellen Kommunikationsvorrichtungen 40, 40', 40", 40"', 40"" sind dazu vorgesehen, Prozessparameter des jeweils zugeordneten Dentalgeräts 12, 12', 12", 12'", 12"" abhängig von den mittels der Sensoreinheit 46 erfassten Umgebungsparametern anzupassen. Alternativ oder zusätzlich können die Umgebungsparameter dabei auch von einer Sensoreinheit des Bedienerendgeräts 18 und/oder von einer externen Quelle stammen. Es ist beispielsweise denkbar, dass die Umgebungsparameter von Smart-Home-Geräten bezogen werden. Alternativ oder zusätzlich wäre denkbar, dass die Umgebungsparameter aus dem Internet 72, wie beispielsweise von Wetterdiensten, bezogen werden. Die erste universelle Kommunikationsvorrichtung 40 ist beispielsweise dazu vorgesehen, eine Mischgeschwindigkeit und/oder eine Mischdauer des ersten Dentalgeräts 12 abhängig von der mittels der Sensoreinheit 46 erfassten Umgebungstemperatur und/oder Umgebungsfeuchtigkeit anzupassen. So kann insbesondere unabhängig von einer sich verändernden Umgebungstemperatur und/oder Umgebungsfeuchtigkeit ein optimales Mischergebnis in der Kapsel gewährleistet werden. Ferner wäre denkbar, dass die universellen Kommunikationsvorrichtungen 40, 40', 40", 40'", 40"" jeweils einen Aktor aufweisen.

Zudem sind die Kommunikationsvorrichtungen 40, 40', 40", 40"', 40"" dazu vorgesehen, die Sensoren 48 der Dentalgeräte 12, 12', 12", 12"', 12"" abhängig von einem erfassten Umgebungsparameter zu kalibrieren. Die universellen Kommunikationsvorrichtungen 40, 40', 40", 40'", 40"" sind dazu vorgesehen, kontinuierlich einen der Sensoren 48 des jeweils gekoppelten Dentalgeräts 12, 12', 12", 12"', 12"" abhängig von einem Standort sowie abhängig von Umgebungsparametern zu kalibrieren. Die Sensoren 48 sind dabei insbesondere von einem einfachen Sensor gebildet, welcher beispielsweise eine ortsabhängige und/oder temperaturabhängige und/oder feuchtigkeitsabhängige Ungenauigkeit aufweist. Eine Ungenauigkeit der Sensoren 48 wird mittels einer Kalibrierung der Sensoren 48 mittels erfassten Umgebungsparametern der universellen Kommunikationsvorrichtungen 40, 40', 40", 40"', 40"" ausgeglichen. Hierdurch ist insbesondere ein Einsatz kostengünstiger Sensoren möglich. Alternativ oder zusätzlich kann eine Anpassung von Prozessparametern über Daten aus dem Internet 72 und/oder erfassten Umgebungsparametern erfolgen. Es kann insbesondere ein Algorithmus und/oder eine künstliche Intelligenz dazu genutzt werden, die Prozesse der Dentalgeräte 12, 12', 12", 12'", 12"" an eine aktuelle Situation, insbesondere mittels Daten aus dem Internet 72 und/oder mittels erfassten Umgebungsparametern, anzupassen. Es ist insbesondere denkbar, dass auf Sensoren in den Dentalgerät 12, 12', 12", 12"', 12"" zumindest teilweise verzichtet werden kann.

Ferner weisen die universellen Kommunikationsvorrichtungen 40, 40', 40", 40"', 40"" jeweils eine Bedieneinheit 50 zu einer kontaktlosen Steuerung zumindest einer Funktion des jeweils gekoppelten Dentalgeräts 12, 12', 12", 12"', 12"", insbesondere mittels Gesten, auf. In der Figur 1 ist der Übersichtlichkeit halber lediglich die Bedieneinheit 50 der ersten universellen Kommunikationsvorrichtung 40 dargestellt. Die Bedieneinheit 50 weist ein optisches Erfassungselement zu einer Erfassung zumindest einer kontaktlosen Bedienung der Bedieneinheit 50 auf. Das optische Erfassungselement ist dazu vorgesehen, zumindest eine Kenngröße eines Bedieners 20 zu erfassen. Die mittels der Bedieneinheit 50 erfasste Kenngröße ist zu einer Auswertung an die Recheneinheit 44 übertragbar. Die Recheneinheit 44 ist dazu vorgesehen, die Kenngröße auszuwerten und daraus einen Bedienbefehl abzuleiten. Mittels der Bedieneinheit 50 ist eine als Geste, als Mimik und/oder als Bewegung eines Bedieners 20 ausgebildete Kenngröße erfassbar. Das optische Erfassungselement ist dazu von einer Kamera gebildet, die zu einer Erfassung der zumindest einen Geste des Bedieners 20 und/oder zumindest einer Bewegung des Bedieners 20 vorgesehen ist. Vorzugsweise ist das optische Erfassungselement zu einer Erfassung der zumindest einen Bewegung des Bedieners 20 vorgesehen, wobei die Bewegung des Bedieners 20 von der Recheneinheit 44 zu einer Auswertung, welcher Geste die erfasste Bewegung des Bedieners 20 entspricht, verarbeitbar ist. Das optische Erfassungselement ist direkt an der jeweiligen universellen Kommunikationsvorrichtung 40, 40', 40", 40"', 40"" angeordnet. In einer alternativen Ausgestaltung wäre jedoch auch denkbar, dass das optische Erfassungselement in dem externen Bedienerendgerät 18 integriert ist oder an einer externen Einheit angeordnet ist.

Über die Bedieneinheit 50 sind verschiedene Funktionen der Dentalgeräte 12, 12', 12", 12"', 12"" steuerbar. Die Funktionen und die damit verbundenen Gesten sind dabei insbesondere von dem jeweiligen Dentalgerät 12, 12', 12", 12"', 12"" anhängig. Vorzugsweise kann mittels der Bedieneinheit 50 zumindest ein Betrieb des Dentalgeräts 12, 12', 12", 12'", 12"" unterbrochen werden. Insbesondere können mittels des Bedienerendgeräts 18, insbesondere mittels einer Applikation, verschiedene Gesten mit verschiedenen Gerätefunktionen verknüpft werden. Es kann insbesondere zu jedem Dentalgerät 12, 12', 12", 12"', 12"" jeder gespeicherten Geste eine Funktion des Dentalgeräts 12, 12', 12", 12"', 12"" zugeteilt werden.

Des Weiteren sind die universellen Kommunikationsvorrichtungen 40, 40', 40", 40"', 40"" mittels der jeweiligen Kommunikationseinheit 16 zu einer drahtlosen Kommunikation mit Fremdgeräten 52, 54, 56 einer Netzwerkumgebung 58 vorgesehen. Die universellen Kommunikationsvorrichtungen 40, 40', 40", 40"', 40"" können über die jeweilige Kommunikationseinheit 16 sowohl mit weiteren Dentalgeräten 12, 12', 12", 12"', 12"" als auch mit Fremdgeräten 52, 54, 56 kommunizieren. Es sind verschiedene, einem Fachmann als sinnvoll erscheinende Fremdgeräte 52, 54, 56 denkbar, wie beispielsweise Smart-Home-Geräte, Wetterstationen. Ferner ist denkbar, dass zumindest eines der Fremdgeräte 52, 54, 56 von einer Kamera, wie insbesondere von einer Überwachungskamera und/oder einer Digitalkamera, gebildet ist. In der Laborumgebung 32 ist beispielhaft ein erstes Fremdgerät 52 angeordnet, welches von einer CAD/CAM-Fräsmaschine gebildet ist. Ferner ist in der Laborumgebung 32 ein zweites Fremdgerät 54 angeordnet, welches von einem Aquastoppventil gebildet ist. Des Weiteren ist in der Laborumgebung 32 ein drittes Fremdgerät 56 angeordnet, welches von einem Smart-Home-System, wie beispielsweise einem Amazon Alexa Smart-Home-System und/oder einem Google Smart-Home-System, gebildet ist. Die Fremdgeräte 52, 54, 56 verfügen jeweils über eine eigenständige nicht weiter dargestellte Kommunikationseinheit zu einer Kommunikation mit den universellen Kommunikationsvorrichtungen 40, 40', 40", 40"', 40"" und sind in ein Netzwerk der Laborumgebung 32 eingebunden. Die Fremdgeräte 52, 54, 56 sind über einen Router 62 der Netzwerkumgebung 58 mit den universellen Kommunikationsvorrichtungen 40, 40', 40", 40"', 40"" gekoppelt. Die universellen Kommunikationsvorrichtungen 40, 40', 40", 40"', 40"" sind dazu vorgesehen, Daten von den Fremdgeräten 52, 54, 56 zu beziehen und/oder mit den Fremdgeräten 52, 54, 56 zu kommunizieren.

Ferner sind die universellen Kommunikationsvorrichtungen 40, 40', 40", 40"', 40"" zu einer Erfassung von Sensor- und/oder Prozessdaten der Fremdgeräte 52, 54, 56 der Netzwerkumgebung 58 vorgesehen ist. Die Sensor- und/oder Prozessdaten der Fremdgeräte 52, 54, 56 können zu einer direkten Steuerung und/oder Regelung des jeweils zugeordneten Dentalgeräts 12, 12', 12", 12"', 12"" genutzt werden. Insbesondere kann das jeweilige Dentalgerät 12, 12', 12", 12"', 12"" mittels der Fremdgeräte 52, 54, 56 funktional erweitert werden, wobei eine Kommunikation zwischen dem Fremdgerät 52, 54, 56 und den Dentalgeräten 12, 12', 12", 12"', 12"" über die universellen Kommunikationsvorrichtungen 40, 40', 40", 40"', 40"" erfolgt. Es ist insbesondere denkbar, dass die Dentalgeräte 12, 12', 12", 12"', 12"" beispielsweise bei einem Signal des Fremdgeräts 52, 54, 56, wie insbesondere des Aquastoppventils und/oder des Nährungsschalters, über die jeweilige universelle Kommunikationsvorrichtung 40, 40', 40", 40'", 40"" gestartet oder gestoppt werden. Die fünfte universelle Kommunikationsvorrichtung 40"" ist beispielsweise dazu vorgesehen, bei einem Signal des zweiten Fremdgeräts 54 das fünfte Dentalgerät 12"" zu aktivieren oder zu deaktivieren. Die zweite universelle Kommunikationsvorrichtung 40' ist beispielsweise dazu vorgesehen, bei einer Aktivierung des ersten Fremdgeräts 52 das zweite Dentalgerät 12' zu aktivieren oder eine Saugleistung an den zusätzlichen Verbraucher, und zwar das erste Fremdgerät 52, automatisch anzupassen. Dazu ruft die zweite universelle Kommunikationsvorrichtung 40' insbesondere kontinuierlich einen Status des ersten Fremdgeräts 52 ab.

Des Weiteren weist das Dentalsystem 10 das Bedienerendgerät 18 auf. Das Bedienerendgerät 18 ist einem Bediener 20 zugeordnet. Jeder Bediener 20 weist insbesondere ein Bedienerendgerät 18 auf. Mittels des Bedienerendgeräts 18 wird der Bediener 20 identifiziert. Es wäre jedoch auch denkbar, dass ein zentrales Bedienerendgerät 18 oder an jedem Dentalgerät 12, 12', 12", 12"', 12"" ein Bedienerendgerät 18 vorgesehen ist, wobei sich der Bediener 20 jeweils an dem Bedienerendgerät 18 zu einer Identifizierung anmelden muss. Das Bedienerendgerät 18 ist beispielhaft von einem Smartphone gebildet. Es wäre jedoch auch eine andere, einem Fachmann als sinnvoll erscheinende Ausgestaltung denkbar. Auf dem Bedienerendgerät 18 ist insbesondere eine Applikation für das Dentalsystem 10 installiert und ausgeführt. Die Applikation kann insbesondere manuell von einem Bediener 20 von einem App-Store heruntergeladen werden. Die Applikation weist insbesondere gewisse Funktionen unabhängig von den eingebundenen Dentalgeräten 12, 12', 12", 12"', 12"" auf. Abhängig von den eingebundenen Dentalgeräten 12, 12', 12", 12"', 12"" werden dann einzelnen eingebundenen Dentalgeräten 12, 12', 12", 12"', 12"" zugeordnete Funktionen freigeschaltet, sowie Synergiefunktionen, welche durch eine Kombination von zumindest zwei eingebundenen Dentalgeräten 12, 12', 12", 12'", 12"" freigeschaltet werden.

In die Applikation ist insbesondere zudem ein Online-Shop integriert. Es können insbesondere direkt Werkzeuge, Ersatzteile und/oder Verbrauchsmaterialen bei dem Dentalgerätehersteller bestellt werden. Zudem ist auch denkbar, dass direkt Bestellungen ausgelöst werden, wenn beispielsweise Teile am Ende ihrer Nutzungsdauer sind oder Verbrauchsmaterialien zur Neige gehen. Es kann insbesondere abhängig von Sensordaten eines der Dentalgeräte 12, 12', 12", 12'", 12"" über das Bedienerendgerät 18 selbsttätig eine Online-Bestellung ausgelöst werden.

Das Bedienerendgerät 18 ist über die Kommunikationseinheiten 16 der universellen Kommunikationsvorrichtungen 40, 40', 40", 40"', 40"" zu einem Datenaustausch mit den Dentalgeräten 12, 12', 12", 12"', 12"" vorgesehen. Die Dentalgeräte 12, 12', 12", 12"', 12"" mit den universellen Kommunikationsvorrichtungen 40, 40', 40", 40"', 40"" werden über das Bedienerendgerät 18 in die Netzwerkumgebung 58 eingebunden. Die Dentalgeräte 12, 12', 12", 12"', 12"" sind jeweils über eine der universellen Kommunikationsvorrichtungen 40, 40', 40", 40"', 40"" in die Netzwerkumgebung 58 eingebunden. Die universellen Kommunikationsvorrichtungen 40, 40', 40", 40"', 40"" sind lediglich mit der lokalen Netzwerkumgebung 58 verbunden, die universellen Kommunikationsvorrichtungen 40, 40', 40", 40"', 40"" sind insbesondere nicht mit dem Internet 72 verbunden und/oder zu einer Kommunikation mit einer externen Servereinheit 24 oder dergleichen vorgesehen. Die universellen Kommunikationsvorrichtungen 40, 40', 40", 40"', 40"" fungieren in der Netzwerkumgebung 58 jeweils als Server, insbesondere eines Client-Server-Modells, während das Bedienerendgerät 18 als Client in der Netzwerkumgebung 58 fungiert. Die universellen Kommunikationsvorrichtungen 40, 40', 40", 40'", 40"" können daher insbesondere lediglich auf Anfragen des Bedienerendgeräts 18 reagieren. Das Bedienerendgerät 18 kann dagegen als Client einen Dienst bei der als Server fungierenden universellen Kommunikationsvorrichtung 40, 40', 40", 40"', 40"" anfordern, die diesen Dienst bereitstellt. Eine Kommunikation zwischen dem Bedienerendgerät 18 und den universellen Kommunikationsvorrichtungen 40, 40', 40", 40"', 40"" ist daher insbesondere lediglich lokal möglich. Das Bedienerendgerät 18 ist zu einer Verbindung mit dem Internet 72 vorgesehen. Insbesondere ist das Bedienerendgerät 18 zu einer Verbindung mit der Servereinheit 24 vorgesehen. Die Servereinheit 24 ist von einem externen Server gebildet. Die Servereinheit 24 ist beispielhaft von einer Cloud gebildet. Die Servereinheit 24 bildet ein Teil des Dentalsystems 10. Es wäre jedoch auch denkbar, dass die universellen Kommunikationsvorrichtungen 40, 40', 40", 40"', 40"" zu einem direkten Internetzugriff vorgesehen sind und/oder dass die universellen Kommunikationsvorrichtungen 40, 40', 40", 40'", 40"" sich nicht als Server in der Netzwerkumgebung 58 befindet, sondern als Client, insbesondere gemäß eines Client-Server-Modells.

Eine Kopplung der universellen Kommunikationsvorrichtungen 40, 40', 40", 40"', 40"" mit der Netzwerkumgebung 58 erfolgt insbesondere über das Bedienerendgerät 18. Die universellen Kommunikationsvorrichtungen 40, 40', 40", 40"', 40"" dienen bei einer Initialisierung insbesondere als Access Point und werden über die Applikation des Bedienerendgeräts 18 mit der lokalen Netzwerkumgebung 58, insbesondere einem lokalen WLAN, verbunden. Die Dentalgeräte 12, 12', 12", 12"', 12"" mit den universellen Kommunikationsvorrichtungen 40, 40', 40", 40"', 40"" sind nach einer Initialisierung jeweils Server, insbesondere gemäß eines Client-Server-Modells, des Routers 62. Ferner können die Dentalgeräte 12, 12', 12", 12"', 12"" und die universellen Kommunikationsvorrichtungen 40, 40', 40", 40"', 40"", insbesondere manuell durch den Bediener 20 über das Bedienerendgerät 18, über ihre Seriennummer registriert werden. Über eine Registrierung erfolgt insbesondere auf einer zentralen Datenbank 64 eine Verknüpfung zwischen dem Bediener 20, der jeweiligen universellen Kommunikationsvorrichtung 40, 40', 40", 40"', 40"" und dem jeweiligen Dentalgerät 12, 12', 12", 12'", 12"". Der Bediener 20, die jeweilige universelle Kommunikationsvorrichtung 40, 40', 40", 40'", 40"" und das jeweilige Dentalgerät 12, 12', 12", 12'", 12"" bilden auf der zentralen Datenbank 64 einen gemeinsamen Datensatz aus. Die zentrale Datenbank 64 ist insbesondere von einer Datenbank 64 des Dentalgeräteherstellers gebildet. Dadurch kann insbesondere eine direkte Anbindung es Dentalgeräteherstellers an den Bediener 20 erreicht werden.

Das Bedienerendgerät 18 ist dazu vorgesehen, dem Bediener 20 zugeordnete Profilparameter für eines der Dentalgeräte 12, 12', 12", 12'", 12"" zu erfassen. Das Bedienerendgerät 18 erfasst dazu, insbesondere über einen Nährungssensor und/oder eine manuelle Eingabe durch den Bediener 20, an welchem der Dentalgeräte 12, 12', 12", 12'", 12"" der Bediener 20 aktuell arbeitet und/oder arbeiten möchte. Das Bedienerendgerät 18 ist dazu vorgesehen, dem Bediener 20 zugeordnete Profilparameter für das Dentalgerät 12, 12', 12", 12'", 12"", an welchem der Bediener 20 arbeitet, von der Servereinheit 24 abzurufen. Es wäre jedoch auch denkbar, dass die Profilparameter direkt auf einem internen Speicher des Bedienerendgeräts 18 hinterlegt sind. Das Bedienerendgerät 18 ist zu einer Anpassung von Prozessparametern des jeweiligen Dentalgeräts 12, 12', 12", 12'", 12"" abhängig von den erfassten Profilparameter vorgesehen. Die Prozessparameter und die Profilparameter sind dabei insbesondere einem gemeinsamen Datenprofil zugeordnet. Die Prozessparameter umfassen insbesondere ein oder mehrere Einstellungen und/oder Konfigurationen für die Dentalgeräte 12, 12', 12", 12"', 12"". Die Einstellungen und/oder Konfigurationen können dabei zwischen den Dentalgeräten 12, 12', 12", 12'", 12"" abweichen. Die Prozessparameter sind mit den Profilparametern gekoppelt. Insbesondere werden die Prozessparameter von den Profilparametern abgeleitet. Abhängig von den ermittelten Profilparametern werden insbesondere die Prozessparameter bestimmt und entsprechend in dem jeweiligen Dentalgerät 12, 12', 12", 12"', 12"" eingestellt. Die Profilparameter können dabei insbesondere zumindest teilweise unabhängig von den Dentalgeräten 12, 12', 12", 12"', 12"" sein und beispielsweise Vitalwerte, allgemeine Kenntniswerte, Geräuschempfindlichkeit, Befugnisdaten oder dergleichen umfassen. Mittels der allgemein gültigen Profilparameter können dann wiederum für jedes der Dentalgeräte 12, 12', 12", 12"', 12"" Prozessparameter abgeleitet werden, oder dass der Bediener 20 bereits davor an dem entsprechenden Dentalgerät 12, 12', 12", 12'", 12"" gearbeitet hat.

Bei dem dritten Dentalgerät 12" wäre hier beispielsweise denkbar, dass auf dem Bedienerendgerät 18 jeweils Benutzer- und Materialprofile hinterlegt sind, welche einen Belichtungsmodus, eine Belichtungshelligkeit oder dergleichen für das dritte Dentalgerät 12" vorgeben. Ferner kann durch die Anmeldung des Bedieners 20 am Dentalgerät 12" eine automatisierte Personifizierung von Bildern oder dergleichen erreicht werden. Es kann insbesondere eine automatisierte Zuordnung von durchgeführten Arbeiten erreicht werden.

Bei dem vierten Dentalgerät 12'" wäre hier beispielsweise denkbar, dass auf dem Bedienerendgerät 18 jeweils Benutzer- und Materialprofile hinterlegt sind, welche eine Farbtemperatur, eine Belichtungshelligkeit, eine Wellenlänge oder dergleichen für das vierte Dentalgerät 12'" vorgeben. Ferner könnte insbesondere ein Nachtmodus vorgesehen sein, bei welchem mit einem reduzierten Blauanteil beleuchtet wird.

Ferner ist das Bedienerendgerät 18 dazu vorgesehen, abhängig von einem erfassten Bediener 20, Energieparameter des Dentalgeräts 12, 12', 12", 12'", 12"", insbesondere des Dentalgeräts 12, 12', 12", 12"', 12"", an welchem der Bediener 20 aktuell arbeitet, über die Kommunikationseinheit 16 der jeweiligen universellen Kommunikationsvorrichtung 40, 40', 40", 40"', 40"" einzustellen. Das Bedienerendgerät 18 ist dazu vorgesehen, abhängig von einem erfassten Bediener 20 sowie dessen Nutzerverhalten ein Energiemanagement des Dentalgeräts 12, 12', 12", 12"', 12"", an welchem der Bediener 20 aktuell arbeitet, über die Kommunikationseinheit 16 der jeweiligen universellen Kommunikationsvorrichtung 40, 40', 40", 40"', 40"" anzupassen. Mittels des Bedienerendgeräts 18 wird zumindest ein ECO-Modus des Dentalgeräts 12, 12', 12", 12'", 12"", an welchem der Bediener 20 aktuell arbeitet, über die Kommunikationseinheit 16 der jeweiligen universellen Kommunikationsvorrichtung 40, 40', 40", 40'", 40"" gesteuert, insbesondere aktiviert und/oder deaktiviert. Ferner können dem Bediener 20 ECO-Empfehlungen ausgegeben werden, die einen Bediener 20 bei einer energieeffizienten Nutzung des Dentalgeräts 12, 12', 12", 12"', 12"" unterstützen. In dem ECO-Modus kann beispielsweise mittels der Energieparameter eine Ausgabeleistung reduziert, ein Regelverhalten verlangsamt und/oder ein Stand-by-Verhalten verändert werden. Der ECO-Modus ist insbesondere individuell an den Bediener 20 angepasst, wobei insbesondere Energieparameter und/oder Regelparameter verändert werden, die einen Betrieb des Bedieners 20 nicht beeinflussen. Es werden daher für jeden Bediener 20 individuell die Energieparameter und/oder Regelparameter verändert, die bei der Arbeitsweise des Bedieners 20 keinen störenden Einfluss haben. Das Bedienerendgerät 18 ist hierfür dazu vorgesehen, die Energieparameter abhängig von einem mittels der Dentalgeräte 12, 12', 12", 12"', 12"" erfassten und mittels des Bedienerendgeräts 18 gespeicherten Nutzerverhaltens des Bedieners 20 festzulegen. Das Bedienerendgerät 18 ist dazu vorgesehen, ein Nutzerverhalten des Bedieners 20 zu dokumentieren und auszuwerten. Das Bedienerendgerät 18 verfügt dafür über einen Auswertealgorithmus und/oder eine künstliche Intelligenz, welche/r abhängig von dem erfassten Nutzerverhalten optimierte Energieparameter für jedes der Dentalgeräte 12, 12', 12", 12'", 12"" ermittelt und insbesondere auf das jeweilige Dentalgerät 12, 12', 12", 12'", 12"" überträgt. Das Nutzerverhalten des Bedieners 20 wird dabei während eines Betriebs ständig von dem Dentalgerät 12, 12', 12", 12'", 12"", an welchem der Bediener 20 aktuell arbeitet, erfasst und über die jeweilige universelle Kommunikationsvorrichtung 40, 40', 40", 40'", 40"" auf das Bedienerendgerät 18 übertragen. Die Daten des Nutzerverhaltens können ferner auch auf dem Dentalgerät 12, 12', 12", 12'", 12"" zwischengespeichert werden, insbesondere sollte keine Verbindung zu dem Bedienerendgerät 18 vorliegen. Die universelle Kommunikationsvorrichtung 40, 40', 40", 40"', 40"" ist insbesondere lediglich zu einer Datenübertragung und nicht zu einer Zwischenspeicherung der Daten vorgesehen. Es wäre jedoch auch denkbar, dass die jeweilige universelle Kommunikationsvorrichtung 40, 40', 40", 40"', 40"" einen eigenen Speicher zu einer Zwischenspeicherung von Daten, insbesondere Nutzerdaten, aufweist. Das Bedienerendgerät 18 kann das Nutzerverhalten auf einem lokalen Speicher oder auf der Servereinheit 24 speichern.

Zudem ist das Bedienerendgerät 18 dazu vorgesehen, mittels der Dentalgeräte 12, 12', 12", 12"', 12"" erfasste Nutzungsdaten eines Bedieners 20 auszuwerten und dem Bediener 20 Handlungsempfehlungen, insbesondere zu einer Prozessoptimierung, auszugeben. Das Bedienerendgerät 18 ist dazu vorgesehen, die Nutzungsdaten bzw. das Nutzerverhalten des Bedieners 20 zu dokumentieren und auszuwerten, wobei das Bedienerendgerät 18 über einen Auswertealgorithmus und/oder eine künstliche Intelligenz verfügt, welche/r abhängig von dem erfassten Nutzerverhalten optimierte Prozessvorgänge und/oder Prozessparameter für jedes der Dentalgeräte 12, 12', 12", 12"', 12"" ermittelt. Daraufhin werden dem Bediener 20 die optimierten Prozessvorgänge in Form von Handlungsempfehlungen ausgegeben. Die Handlungsempfehlungen können dabei insbesondere das Anpassen von Abläufen, die Verwendung von Produkten oder dergleichen beinhalten. Insbesondere ist denkbar, dass die Handlungsempfehlungen und/oder die Erfassung der Nutzungsdaten eines Bedieners 20 geräteübergreifend, also insbesondere bei mehreren Dentalgeräten 12, 12', 12", 12"', 12"" einer Laborumgebung 32 und/oder einer Praxisumgebung, erfolgen. Vorzugsweise können die Handlungsempfehlungen dadurch insbesondere auch geräteübergreifende Prozesse betreffen.

Das Bedienerendgerät 18 ist dazu vorgesehen, das mittels der Dentalgeräte 12, 12', 12", 12"', 12"" erfasste Nutzerverhalten auszuwerten und auf einer Speichereinheit 22 zu hinterlegen. Die Speichereinheit 22 kann insbesondere als ein Teil des Bedienerendgeräts 18 ausgebildet sein sowie von einer externen Speichereinheit der Servereinheit 24 gebildet sein. Beispielhaft weist das Bedienerendgerät 18 die Speichereinheit 22 auf. Die Speichereinheit 22 ist beispielhaft von einem internen Speicher des Bedienerendgeräts 18 gebildet. Ferner ist das Bedienerendgerät 18 dazu vorgesehen, ein gespeichertes Nutzerverhalten auf die Servereinheit 24 zu übertragen. Das Bedienerendgerät 18 ist mittels einer Internetverbindung, insbesondere mittels einer Mobilfunkverbindung und/oder der Internetverbindung des Routers 62, mit der Servereinheit 24 verbunden. Die Nutzerdaten werden dadurch auf einer zentralen Servereinheit 24 gespeichert und können so unabhängig von einem Standort abgerufen werden. Die Servereinheit 24 ist dazu vorgesehen, bedienerübergreifend Daten eines Nutzerverhaltens auszuwerten und daraus optimierte Prozessparameter abzuleiten. Die Nutzerdaten werden dazu auf der zentralen Servereinheit 24 gesammelt und ausgewertet. Dabei werden insbesondere optimierte Prozessparameter und/oder Prozessabläufe für die Dentalgeräte 12, 12', 12", 12"', 12"" abgeleitet. Die Erfassung des Nutzerverhaltens erfolgt dabei insbesondere bedienerübergreifend und laborumgebungsübergreifend. Die Servereinheit 24 ist dazu vorgesehen, die Daten eines Nutzerverhaltens laborumgebungsübergreifend auszuwerten und daraus für alle Bediener 20 optimierte Prozessparameter und/oder Handlungsempfehlungen abzuleiten. Es wäre insbesondere denkbar, dass ein Nutzerverhalten eines Bedieners 20 mit vorteilhaften Prozessparametern und/oder Prozessabläufen dazu genutzt wird, anderen Nutzern Handlungsempfehlungen oder verbesserte Prozessparameter auszugeben. Die Erfahrungswerte von tausenden Nutzern eines Dentalgeräts können so insbesondere einem Bediener 20 zu Gute kommen. Es wäre insbesondere denkbar, dass so jeder Bediener 20 von anderen Bedienern 20 lernen kann. Es wäre insbesondere denkbar, dass die Optimierung von Prozessparametern und/oder Prozessabläufen und/oder Handlungsempfehlungen mit einem Bezahldienst gekoppelt sind, sodass für die Optimierung von Prozessparametern und/oder Prozessabläufen und/oder für Handlungsempfehlungen bezahlt werden kann und/oder muss.

Des Weiteren ist das Bedienerendgerät 18 dazu vorgesehen, über die Kommunikationseinheit 16 der jeweiligen universellen Kommunikationsvorrichtung 40, 40', 40", 40"', 40"" eines der Dentalgeräte 12, 12', 12", 12"', 12"" abhängig von Parametern eines andere Dentalgeräts 12, 12', 12", 12'", 12"" anzusteuern. Durch die Kopplung mehrerer, insbesondere verschiedener, Dentalgeräte 12, 12', 12", 12"', 12"" mit dem Bedienerendgerät 18 werden Synergiefunktionen zwischen den Dentalgeräten 12, 12', 12", 12'", 12"" erzeugt. Das Bedienerendgerät 18 ist zu einer voneinander abhängigen Ansteuerung der Dentalgeräte 12, 12', 12", 12'", 12"" vorgesehen. Das Bedienerendgerät 18 ist dazu vorgesehen, abhängig von einem Betriebszustand eines der Dentalgeräte 12, 12', 12", 12"', 12"" ein anderes Dentalgerät 12, 12', 12", 12"', 12"" anzusteuern. So kann beispielsweise das zweite Dentalgerät 12' abhängig von einem Betriebszustand der anderen Dentalgeräte 12, 12", 12'", 12"" aktiviert und/oder eine Saugleistung angepasst werden. Hierdurch kann insbesondere eine Saugleistung des zweiten Dentalgeräts 12' an die Verbraucher angepasst werden. Das Bedienerendgerät 18 kann über die Kommunikationseinheit 16 der universellen Kommunikationsvorrichtung 40, 40', 40", 40"', 40"" zu einer gleichzeitigen Ansteuerung mehrerer Dentalgeräte 12, 12', 12", 12"', 12"" vorgesehen sein.

Ferner ist das Bedienerendgerät 18 dazu vorgesehen, über die Kommunikationseinheit 16 einen Wartungszugriff, insbesondere einen Wartungsfernzugriff, auf die Dentalgeräte 12, 12', 12", 12"', 12"" bereitzustellen. Über das Bedienerendgerät 18 kann ein Wartungszugriff auf die Dentalgeräte 12, 12', 12", 12"', 12"" über das Internet 72 hergestellt werden. Der Wartungszugriff dient für einen Servicemitarbeiter zu einer Wartung der Dentalgeräte 12, 12', 12", 12"', 12"" und/oder zu einem externen Aufspielen von Updates. Über den Wartungszugriff kann von einer externen Servicestelle aus auf die Steuer- und/oder Regeleinheit 14 der Dentalgeräte 12, 12', 12", 12"', 12"" zugegriffen werden. Über den Wartungszugriff können von einer externen Servicestelle aus Parameter der Dentalgeräte 12, 12', 12", 12'", 12"" angepasst werden. Ferner kann über den Wartungszugriff auf ein Fehlerprotokoll oder dergleichen der Dentalgeräte 12, 12', 12", 12"', 12"" zugegriffen werden.

Zudem ist das Bedienerendgerät 18 in zumindest einem Betriebszustand zu einer Steuerung eines der Dentalgeräte 12, 12', 12", 12'", 12"" über die jeweilige Kommunikationsvorrichtung 40, 40', 40", 40"', 40"" vorgesehen. Über das Bedienerendgerät 18 können Einstellungen an den Dentalgeräten 12, 12', 12", 12"', 12"" vorgenommen werden. Das Bedienerendgerät 18 führt dazu mittels der Applikation eine Benutzeroberfläche für jedes der Dentalgeräte 12, 12', 12", 12"', 12"" aus. Die Benutzeroberflächen können insbesondere je nach Dentalgerät 12, 12', 12", 12"', 12"" variieren. Das Bedienerendgerät 18 verfügt dafür über eine Bedien- und Anzeigeeinheit 66. Die Bedien- und Anzeigeeinheit 66 ist beispielhaft von einem Touchscreen des Bedienerendgeräts 18 gebildet. Ferner kann das Bedienerendgerät 18 zu einer selbsttätigen Steuerung der Dentalgeräte 12, 12', 12", 12"', 12"" vorgesehen sein. Vorzugsweise können über das Bedienerendgerät 18 insbesondere erweiterte Einstellmöglichkeiten bereitgestellt werden, welche direkt an dem jeweiligen Dentalgerät 12, 12', 12", 12'", 12"" nicht möglich sind.

Beispielsweise wäre denkbar, dass bei dem vierten Dentalgerät 12'" über das Bedienerendgerät 18 eine Boost-Funktion für helleres Licht und/oder ein Nachtmodus, bei welchem mit einem reduzierten Blauanteil beleuchtet wird, freigeschaltet und aktiviert werden kann. Ferner wäre denkbar, dass bei dem fünften Dentalgerät 12"" über das Bedienerendgerät 18 ein Slow-Steam-Modus freigeschaltet und aktiviert werden kann. In dem Slow-Steam-Modus wird insbesondere der Druck in einem Kessel des fünften Dentalgeräts 12"" abgesenkt. Der Slow-Steam-Modus kann beispielsweise aus ökologischen Gründen genutzt werden, um einen Energiebedarf des fünften Dentalgeräts 12"" zu reduzieren. Alternativ oder zusätzlich kann der Slow-Steam-Modus zu einer Reduzierung der Abstrahlleistung genutzt werden, beispielsweise bei bestimmten, empfindlichen Restaurationen.

Des Weiteren können über das Bedienerendgerät 18 zudem, insbesondere in Form von Push-Nachrichten, aktuelle Informationen zu den Dentalgeräten 12, 12', 12", 12"', 12"" ausgegeben werden. So können beispielsweise bei dem fünften Dentalgerät 12"" Wartungshinweise, wie insbesondere Informationen über den Verkalkungsgrad und/oder über eine Spülung, ausgegeben werden. Ferner können bei dem fünften Dentalgerät 12"" mittels einer akustischen und/oder visuellen Rückmeldung auf dem Bedienerendgerät 18 eine Betriebsbereitschaft und/oder ein aktueller Status angezeigt werden. Vorzugsweise können die Informationen zu den Dentalgeräten 12, 12', 12", 12"', 12"" von einem Bediener 20 in der Applikation des Bedienerendgeräts 18 abgerufen werden.

Ferner sind die universellen Kommunikationsvorrichtungen 40, 40', 40", 40"', 40"" jeweils dazu vorgesehen, über das Bedienerendgerät 18 Produktinformationen abzurufen und Prozessparameter des Dentalgeräts 12, 12', 12", 12"', 12"" abhängig von abgerufenen Produktinformationen anzupassen. Die Prozessparameter umfassen dabei ein oder mehrere Einstellungen und/oder Konfigurationen für das jeweilige Dentalgerät 12, 12', 12", 12"', 12"". Die Produktinformationen können beispielsweise Mischparameter, wie Mischungsverhältnisse, Mischzeiten oder dergleichen, Viskositäten, Abbindezeiten, Aushärtungsparameter oder dergleichen umfassen. Es sind verschiedene, einem Fachmann als sinnvoll erscheinende Produktinformationen denkbar. Beispielsweise wäre denkbar, dass bei dem ersten Dentalgerät 12 selbsttätig Produktinformationen zu einer aktuell verwendeten Kapsel automatisch abgerufen werden und Prozessparameter des ersten Dentalgeräts 12 abhängig von den abgerufenen Produktinformationen angepasst werden. Eine Erkennung der aktuell verwendeten Kapsel kann beispielsweise automatisiert in dem ersten Dentalgerät 12 mittels eines Sensors erfolgen oder beispielsweise durch Scannen mittels des Bedienerendgeräts 18. Zudem wäre denkbar, dass bei dem vierten Dentalgerät 12'" selbsttätig Produktinformationen zu einer Aushärtung eines verwendeten Materials automatisch abgerufen werden und Prozessparameter des vierten Dentalgeräts 12'" abhängig von den abgerufenen Produktinformationen angepasst werden. Es wird insbesondere ein UV-Lichtanteil und/oder eine Bestrahlungsdauer abhängig von dem verwendeten Material eingestellt.

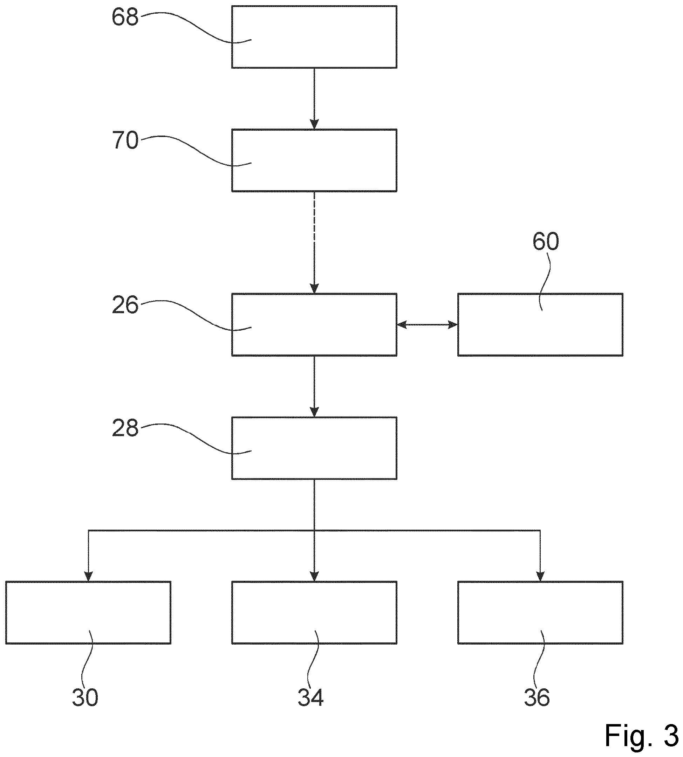

Figur 3 zeigt ein schematisches Ablaufdiagramm eines Verfahrens zu einem Betrieb des Dentalsystems 10.

Vor einem eigentlichen Betrieb den Dentalsystems 10 müssen die Dentalgeräte 12, 12', 12", 12"', 12"" und die universellen Kommunikationsvorrichtungen 40, 40', 40", 40"', 40"" jeweils in die Netzwerkumgebung 58 der Laborumgebung 32 eingebunden werden. Dazu weist das Verfahren einen Verbindungsschritt 68 auf. In dem Verbindungsschritt 68 erfolgt die Kopplung der universellen Kommunikationsvorrichtungen 40, 40', 40", 40"', 40"" und darüber auch der Dentalgeräte 12, 12', 12", 12"', 12"" mit der Netzwerkumgebung 58 über das Bedienerendgerät 18. Die universellen Kommunikationsvorrichtungen 40, 40', 40", 40"', 40"" dienen bei einem ersten Starten und/oder bei einer fehlenden Verbindung zu einer Netzwerkumgebung 58 als Access Point und sind jeweils als WLAN sichtbar. Es kann daher eine direkte WLAN-Verbindung zwischen der jeweiligen universellen Kommunikationsvorrichtung 40, 40', 40", 40"', 40"" und dem Bedienerendgerät 18 hergestellt werden. Anschließend werden die universellen Kommunikationsvorrichtungen 40, 40', 40", 40'", 40"" einzeln über die Applikation des Bedienerendgeräts 18 mit der lokalen Netzwerkumgebung 58, insbesondere einem lokalen WLAN, verbunden. Die Dentalgeräte 12, 12', 12", 12"', 12"" mit den universellen Kommunikationsvorrichtungen 40, 40', 40", 40'", 40"" sind nach einer Initialisierung jeweils Server, insbesondere gemäß eines Client-Server-Modells, des Routers 62. Darauffolgend können die Dentalgeräte 12, 12', 12", 12"', 12"" und die universellen Kommunikationsvorrichtungen 40, 40', 40", 40"', 40"" manuell durch den Bediener 20 über das Bedienerendgerät 18 über ihre Seriennummer in einem Registrierungsschritt 70 registriert werden. Über eine Registrierung erfolgt insbesondere auf einer zentralen Datenbank 64 eine Verknüpfung zwischen dem Bediener 20, der jeweiligen universellen Kommunikationsvorrichtung 40, 40', 40", 40'", 40"" und dem jeweiligen Dentalgerät 12, 12', 12", 12"', 12"". Anschließend kann ein normaler Betrieb des Dentalsystems 10 erfolgen.

Das Verfahren umfasst in einem regulären Betrieb zumindest einen Kopplungsschritt 60, bei dem die universellen Kommunikationsvorrichtungen 40, 40', 40", 40"', 40"" jeweils eine drahtlose Kommunikation mit einem der Fremdgeräte 52, 54, 56 der Netzwerkumgebung 58 zu einem Datenaustausch herstellen. Der Kopplungsschritt 60 wird dabei insbesondere selbsttätig durch die universellen Kommunikationsvorrichtungen 40, 40', 40", 40'", 40"" durchgeführt, wenn ein entsprechendes Fremdgerät 52, 54, 56 erkannt wird. Die Kopplungsschritt 60 kann daher während eines Betriebs mehrfach durchgeführt werden. Es wäre jedoch auch denkbar, dass der Kopplungsschritt 60 manuell durch den Bediener 20, wie insbesondere durch einen Befehl auf dem Bedienerendgerät 18, eingeleitet wird. In dem Kopplungsschritt 60 kann zudem eine Verbindung zwischen den universellen Kommunikationsvorrichtungen 40, 40', 40", 40"', 40"" und damit zwischen den Dentalgeräten 12, 12', 12", 12"', 12"" zu einem Datenaustausch hergestellt werden.

Ferner umfasst das Verfahren in einem regulären Betrieb einen automatischen Anmeldeschritt 26. In dem Anmeldeschritt 26 ruft das Bedienerendgerät 18 dem Bediener 20 zugeordnete Profilparameter für das Dentalgerät 12, 12', 12", 12"', 12"" ab, an welchem der Bediener 20 aktuell arbeitet und/oder plant zu arbeiten. Dazu wird mittels des Bedienerendgeräts 18, welches der Bediener 20 mitführt, ein aktueller Standort innerhalb der Laborumgebung 32 erfasst. Tritt der Bediener 20 an ein Dentalgerät 12, 12', 12", 12"', 12"" heran, werden selbsttätig dem Bediener 20 zugeordnete Profilparameter für das Dentalgerät 12, 12', 12", 12"', 12"" abgerufen. Anschließend werden in dem Anmeldeschritt 26 Prozessparameter des entsprechenden Dentalgeräts 12, 12', 12", 12"', 12"" abhängig von den dem Bediener 20 zugeordneten Profilparametern angepasst. Darauffolgend wird in einem Verfahrensschritt 28 mittels des Bedienerendgeräts 18 abhängig von einem erfassten Bediener 20 zumindest eine Gerätefunktion des Dentalgeräts 12, 12', 12", 12'", 12"", an welchem der Bediener 20 aktuell arbeitet und/oder plant zu arbeiten, freigegeben und/oder gesperrt. Es ist insbesondere denkbar, dass verschiedene Benutzergruppen mit verschiedenen Rechten anlegbar sind. Entsprechende Freigaben des Bedieners 20 für jedes Dentalgerät 12, 12', 12", 12"', 12"" sind auf dem Bedienerendgerät 18 hinterlegt. Darauffolgend kann in einem weiteren Verfahrensschritt 30 das Bedienerendgerät 18 über die Kommunikationseinheit 16 der universellen Kommunikationsvorrichtung 40, 40', 40", 40"', 40"" das Dentalgerät 12, 12', 12", 12"', 12"", an welchem der Bediener 20 aktuell arbeitet, abhängig von einem Status und/oder einem Betriebszustand eines der weiteren Dentalgeräte 12, 12', 12", 12'", 12"" angesteuert werden. Ferner können in einem Optimierungsschritt 34 des Verfahrens dem Bediener 20 über das Bedienerendgerät 18 abhängig von einem mittels des Dentalgeräts 12, 12', 12", 12'", 12"", an welchem der Bediener 20 aktuell arbeitet, erfassten Nutzerverhaltens Prozessoptimierungen angeboten werden. Das Bedienerendgerät 18 wertet dazu ein erfasstes Nutzerverhalten des Bedieners 20 aus. Das Bedienerendgerät 18 verfügt dazu über einen Auswertealgorithmus und/oder eine künstliche Intelligenz, welche/r abhängig von dem erfassten Nutzerverhalten optimierte Prozessvorgänge und/oder Prozessparameter des Dentalgeräts 12, 12', 12", 12'", 12"", an welchem der Bediener 20 aktuell arbeitet, ermittelt. Insbesondere werden dem Bediener 20 die optimierten Prozessvorgänge in Form von Handlungsempfehlungen ausgegeben. Die Handlungsempfehlungen können dabei insbesondere das Anpassen von Abläufen, die Verwendung von Produkten oder dergleichen beinhalten. Insbesondere ist denkbar, dass die Handlungsempfehlungen und/oder die Erfassung der Nutzungsdaten eines Bedieners 20 geräteübergreifend, also insbesondere bei mehreren Dentalgeräten 12, 12', 12", 12"', 12"" der Laborumgebung 32 erfolgen. Des Weiteren können in einem Anpassungsschritt 36 des Verfahrens die zumindest eine universelle Kommunikationsvorrichtung 40 abhängig von einer erfassten Bedienereingabe, einem erfassten Umgebungsparameter und/oder einer erfassten Produktinformation Prozessparameter des Dentalgeräts 12, 12', 12", 12"', 12"", an welchem der Bediener 20 aktuell arbeitet, anpasst werden. In dem Anpassungsschritt 36 werden mittels des Bedienerendgeräts 18 Produktinformationen abgerufen und Prozessparameter des Dentalgeräts 12, 12', 12", 12'", 12"", an welchem der Bediener 20 aktuell arbeitet, abhängig von abgerufenen Produktinformationen angepasst. Die Produktinformationen können beispielsweise Mischparameter, wie Mischungsverhältnisse, Mischzeiten oder dergleichen, Viskositäten, Abbindezeiten, Aushärtungsparameter oder dergleichen umfassen. Es sind verschiedene, einem Fachmann als sinnvoll erscheinende Produktinformationen denkbar. Die Produktinformationen werden von dem Bedienerendgerät 18 über das Internet 72 bezogen.

### Bezugszeichenliste

- 10: Dentalsystem
- 12: Dentalgerät
- 14: Steuer- und/oder Regeleinheit
- 16: Kommunikationseinheit
- 18: Bedienerendgerät
- 20: Bediener
- 22: Speichereinheit
- 24: Servereinheit
- 26: Anmeldeschritt
- 28: Verfahrensschritt
- 30: Verfahrensschritt
- 32: Laborumgebung
- 34: Optimierungsschritt
- 36: Anpassungsschritt
- 38: Datenschnittstelle
- 40: Kommunikationsvorrichtung
- 42: Datenschnittstelle
- 44: Recheneinheit
- 46: Sensoreinheit
- 48: Sensor
- 50: Bedieneinheit
- 52: Fremdgerät
- 54: Fremdgerät
- 56: Fremdgerät
- 58: Netzwerkumgebung
- 60: Kopplungsschritt
- 62: Router
- 64: Datenbank
- 66: Bedien- und Anzeigeeinheit
- 68: Verbindungsschritt
- 70: Registrierungsschritt
- 72: Internet

## Patentansprüche

1. Dentalsystem mit zumindest einem Dentalgerät (12, 12', 12", 12"', 12""), welches zumindest eine Steuer- und/oder Regeleinheit (14) aufweist, die zu einer Steuerung und/oder Regelung zumindest eines Prozesses des Dentalgeräts (12, 12', 12", 12"', 12"") vorgesehen ist, mit zumindest einer mit dem Dentalgerät (12, 12', 12", 12"', 12"") gekoppelten Kommunikationseinheit (16) und mit zumindest einem Bedienerendgerät (18), welches über die Kommunikationseinheit (16) zu einem Datenaustausch mit dem Dentalgerät (12, 12', 12", 12"', 12"") vorgesehen ist,
**dadurch gekennzeichnet, dass**
das Bedienerendgerät (18) dazu vorgesehen ist, dem Bediener (20) zugeordnete Profilparameter für das Dentalgerät (12, 12', 12", 12"', 12"") zu erfassen.

2. Dentalsystem nach Anspruch 1,
**dadurch gekennzeichnet, dass**
das Bedienerendgerät (18) zu einer Anpassung zumindest eines Prozessparameters des Dentalgeräts (12, 12', 12", 12"', 12"") abhängig von dem zumindest einen Profilparameter vorgesehen ist.

3. Dentalsystem nach Anspruch 1 oder 2,
**dadurch gekennzeichnet, dass**
das Bedienerendgerät (18) dazu vorgesehen ist, abhängig von einem erfassten Bediener (20), zumindest einen Energieparameter des Dentalgeräts (12, 12', 12", 12"', 12"") über die Kommunikationseinheit (16) einzustellen.

4. Dentalsystem nach Anspruch 3,
**dadurch gekennzeichnet, dass**
das Bedienerendgerät (18) dazu vorgesehen ist, den Energieparameter abhängig von einem mittels des Dentalgeräts (12, 12', 12", 12"', 12"") erfassten und mittels des Bedienerendgeräts (18) gespeicherten Nutzerverhaltens des Bedieners (20) festzulegen.

5. Dentalsystem nach einem der vorhergehenden Ansprüche,
**dadurch gekennzeichnet, dass**
das Bedienerendgerät (18) dazu vorgesehen ist, mittels des Dentalgeräts (12, 12', 12", 12"', 12"") erfasste Nutzungsdaten eines Bedieners (20) auszuwerten und dem Bediener (20) Handlungsempfehlungen, insbesondere zu einer Prozessoptimierung, auszugeben.

6. Dentalsystem zumindest nach dem Oberbegriff des Anspruchs 1, insbesondere nach einem der vorhergehenden Ansprüche,
**gekennzeichnet durch**
zumindest ein weiteres Dentalgerät (12', 12", 12'", 12""), welches zumindest eine Steuer- und/oder Regeleinheit aufweist, die zu einer Steuerung und/oder Regelung zumindest eines Prozesses des weiteren Dentalgeräts (12', 12", 12"', 12"") vorgesehen ist, mit zumindest einer mit dem weiteren Dentalgerät (12', 12", 12"', 12"") gekoppelten weiteren Kommunikationseinheit, wobei das Bedienerendgerät (18) dazu vorgesehen ist, über die Kommunikationseinheit (16) das Dentalgerät (12) abhängig von zumindest einem Parameter des weiteren Dentalgeräts (12', 12", 12"', 12"") anzusteuern.

7. Dentalsystem nach Anspruch 6,
**dadurch gekennzeichnet, dass**
das Bedienerendgerät (18) über die Kommunikationseinheit (16) und die weitere Kommunikationseinheit zu einer gleichzeitigen Ansteuerung des Dentalgeräts (12) und des weiteren Dentalgeräts (12', 12", 12"', 12"") vorgesehen ist.

8. Dentalsystem zumindest nach dem Oberbegriff des Anspruchs 1, insbesondere nach einem der vorhergehenden Ansprüche,
**dadurch gekennzeichnet, dass**
das Bedienerendgerät (18) dazu vorgesehen ist, ein mittels des zumindest einen Dentalgeräts (12, 12', 12", 12"', 12"") erfasstes Nutzerverhalten auszuwerten und auf einer Speichereinheit (22) zu hinterlegen.

9. Dentalsystem nach Anspruch 8,
**gekennzeichnet durch**
eine Servereinheit (24), wobei das Bedienerendgerät (18) dazu vorgesehen ist, ein gespeichertes Nutzerverhalten auf die Servereinheit (24) zu übertragen.

10. Dentalsystem nach Anspruch 9,
**dadurch gekennzeichnet, dass**
die Servereinheit (24) dazu vorgesehen ist, insbesondere bedienerübergreifend, Daten eines Nutzerverhaltens auszuwerten und daraus optimierte Prozessparameter abzuleiten.

11. Dentalsystem nach einem der vorhergehenden Ansprüche,
**dadurch gekennzeichnet, dass**
das Bedienerendgerät (18) dazu vorgesehen ist, über die Kommunikationseinheit (16) einen Wartungszugriff, insbesondere einen Wartungsfernzugriff, auf das Dentalgerät (12, 12', 12", 12"', 12"") bereitzustellen.

12. Verfahren zu einem Betrieb des Dentalsystems (10) nach einem der vorhergehenden Ansprüche, bei dem in zumindest einem, insbesondere automatischen, Anmeldeschritt (26) das Bedienerendgerät (18) dem Bediener (20) zugeordnete Profilparameter für das Dentalgerät (12, 12', 12", 12'", 12"") abruft.

13. Verfahren nach Anspruch 12,
**dadurch gekennzeichnet, dass**
in dem zumindest einen Anmeldeschritt (26) Prozessparameter des Dentalgeräts (12, 12', 12", 12"', 12"") abhängig von dem zumindest einen, dem Bediener (20) zugeordneten Profilparameter angepasst werden.

14. Verfahren nach Anspruch 12 oder 13,
**dadurch gekennzeichnet, dass**
in zumindest einem Verfahrensschritt (28), insbesondere mittels des Bedienerendgeräts (18), abhängig von einem erfassten Bediener (20) zumindest eine Gerätefunktion des Dentalgeräts (12, 12', 12", 12"', 12"") freigegeben und/oder gesperrt wird.

15. Verfahren nach einem der Ansprüche 12 bis 14,
**dadurch gekennzeichnet, dass**
in zumindest einem Verfahrensschritt (30) das Bedienerendgerät (18) über die Kommunikationseinheit (16) das Dentalgerät (12) abhängig von einem Status und/oder einem Betriebszustand eines weiteren Dentalgeräts (12', 12", 12"', 12""), insbesondere eines weiteren Dentalgeräts (12', 12", 12"', 12"") derselben Laborumgebung (32) und/oder Praxisumgebung, ansteuert.

16. Verfahren nach einem der Ansprüche 12 bis 15,
**dadurch gekennzeichnet, dass**
in zumindest einem Optimierungsschritt (34) dem Bediener (20) über das Bedienerendgerät (18) abhängig von einem mittels des Dentalgeräts (12, 12', 12", 12"', 12"") erfassten Nutzerverhalten Prozessoptimierungen angeboten werden.

17. Verfahren nach einem der Ansprüche 12 bis 16,
**dadurch gekennzeichnet, dass**
in zumindest einem Anpassungsschritt (36) mittels des Bedienerendgeräts (18) Produktinformationen abgerufen werden und Prozessparameter des Dentalgeräts (12, 12', 12", 12"', 12"") abhängig von abgerufenen Produktinformationen angepasst werden.
